# EUROPEAN PATENT SPECIFICATION

(11) **EP 3 884 055 B1**
(45) Date of publication and mention of the grant of the patent: **09.08.2023**
(21) Application number: 19801284.1
(22) Date of filing: 07.11.2019
(51) Int. Cl.: C12N 15/82, C12N 9/12, C12N 9/64, C12N 15/79

(54) **DOUBLE AND INDUCIBLE SUICIDE GENE CONSTRUCT AND ITS USE IN GENE THERAPY**
DOPPELTES UND INDUZIERBARE SUIZIDGENKONSTRUKT UND DESSEN VERWENDUNG IN DER GENTHERAPIE
CONSTRUCTION DE GÈNES SUICIDES DOUBLES ET INDUCTIBLES ET SON UTILISATION DANS LA THÉRAPIE GÉNIQUE

(30) Priority: 07.11.2018 EP 18382792
(43) Date of publication of application: 29.09.2021
(73) Proprietor: Consejo Superior de Investigaciones Científicas (CSIC), 28006 Madrid (ES)
(72) Inventor: BACHILLER PÉREZ, Daniel, 07190 Esporles (Illes Balears) (ES); FLEISCHER, Aarne, 07190 Esporles (Illes Balears) (ES); VALLEJO DIEZ, Sara, 07190 Esporles (Illes Balears) (ES)
(74) Representative: Pons
(86) International application number: PCT/EP2019/080485
(87) International publication number: WO 2020/094764

(56) References cited:
- EP-A1- 3 095 864
- XIAN-RUN LUO ET AL: "Adenovirus-mediated Double Suicide Gene Selectively Kills Gastric Cancer Cells", ASIAN PACIFIC JOURNAL OF CANCER PREVENTION, vol. 13, no. 3, 31 March 2012 (2012-03-31) , pages 781-784, XP055569916, TH ISSN: 1513-7368, DOI: 10.7314/APJCP.2012.13.3.781
- CHUANFENG WU ET AL: "Development of an inducible caspase-9 safety switch for pluripotent stem cell-based therapies", MOLECULAR THERAPY - METHODS & CLINICAL DEVELOP, vol. 1, 1 January 2014 (2014-01-01), page 14053, XP055569710, GB ISSN: 2329-0501, DOI: 10.1038/mtm.2014.53
- AURELIE BEDEL ET AL: "Preventing Pluripotent Cell Teratoma in Regenerative Medicine Applied to Hematology Disorders : Evaluation and Control of Teratoma Risk in Hematology", STEM CELLS TRANSLATIONAL MEDICINE, vol. 6, no. 2, 7 September 2016 (2016-09-07), pages 382-393, XP055569685, US ISSN: 2157-6564, DOI: 10.5966/sctm.2016-0201

## Description

The present invention falls within the field of genetic engineering and gene and cell therapy, specifically within gene constructs or suicide cassettes comprising inducible genes that trigger the cellular death in cells expressing them. Said constructs can therefore be used to induce directed and specific cell death in specific cell populations whose presence poses a risk to the organism. Thus, said suicide constructs represent genetic safety tools in, for example, the treatment and/or prevention of diseases or conditions such as cancer, graft-versus-host disease or transplant rejection. The references to methods for treatment of the human or animal body by surgery or therapy are to be interpreted as references to the compounds, pharmaceutical compositions and medicaments of the present invention for use in those methods.

### STATE OF THE ART

The use of cell therapies in clinical practice entails potential toxicity arising from prior manipulation when culturing the transplanted cells which, once transferred to the receptor organism, may pose a risk. For example, the transplant of hematopoietic cells can trigger graft-versus-host disease and the transplant of stem cells may cause uncontrolled cell proliferation, generating tumors. Accordingly, there is considerable interest in developing genetic safety mechanisms that can be externally controlled and activated, if necessary, so as to enable the selective elimination of transplanted cells in a controlled, conditioned and induced manner, should they represent a health problem for the individual.

Since the effect of the transplanted cells in cell therapies can be prolonged over time or even indefinite, thereby causing the toxicity arising from the treatment to be sudden and/or progressive, safety mechanisms are required to eliminate these transplanted cells in case of appearance of adverse effects. In this regard, it was possible to selectively eliminate cells transferred to organisms through the exogenous activation of cell safety mechanisms expressed recombinantly in said cells and known as "suicide genes".

A suicide gene encodes a molecule that, when activated, triggers the selective destruction of the transplanted cells that express it, thereby preventing cell and/or tissue damage caused by them. The ideal suicide gene would therefore be that which ensures the irreversible elimination of all the cells responsible for the unwanted toxicity and whose effect is harmless to other, non-target cells.

This therapeutic strategy based on the use of suicide genes is not only applicable to transplant cases, but rather has also become a new treatment of interest in cancer therapies. In particular, suicide gene therapy in cancer treatments consists of transfecting the suicide gene to the tumor cells by means of directed vectors so as to produce a specific enzyme that becomes a cytotoxic product once activated, which finally causes the specific death of the tumor cells.

In general, suicide genes can be classified in accordance with their action mechanism into: genes that encode an enzyme that would act as a pro-drug, genes that encode a molecule whose activation depends on its dimerized state and genes that encode a molecule activated by a monoclonal antibody. The ideal agent for activating a suicide gene should be biologically inert, have adequate bioavailability and biodistribution profiles and not have intrinsic toxicity.

In recent years various suicide constructs have been designed including, namely, those based on the herpes simplex virus thymidine kinase (HSV-TK) gene or on the apoptotic activity of caspase 9 in mammals. In both cases, they are harmless proteins constitutively expressed. However, when caspase 9 is provided to its specific activator or TK to its substrate, processes ending in the death in cells expressing them are triggered.

The HSV-TK gene has been used to trigger directed cell death cascade (Ciceri, F., et al., 2009, The lancet oncology, 10(5): p. 489-500). This system is based on the introduction of the TK gen in the cells in constitutive expression format. The product of the gene expression is harmless unless the enzyme substrate is supplied to Ganciclovir (GCV) cells, whose metabolism gives rise to a base analog. The compound is included in the DNA during the replication stage, introducing copy errors that end up limiting cell variability. However, this system has a series of drawbacks that make it inadequate for clinical use since, as can be observed, on the one hand functioning is limited to the proliferative period of cell life. Additionally, in its practical applications it has never achieved the elimination of all the target cells (Xu, F., et al., 2009, Cancer gene therapy, 16(9): p. 723-30). Furthermore, although the HSV-TK/GCV suicide system can be apparently effective as a safety mechanism in certain situations, such as graft-versus-host disease, it generally has a series of drawbacks such as the potential immunogenicity of the HSV-TK viral protein. Additionally, said enzyme requires GCV to activate a medicament, which is a crucial pharmacological agent for treating one of the most common infections in transplant situations, infection by cytomegalovirus. This leads to the unwanted elimination of transduced cell populations when said pharmacological agent is used to treat this virus (Traversari C, Marktel S, Magnani Z, et al., 2007, Blood, 109:4708-4715). Lastly, complete HSV-TK/GCV-mediated cell death takes days to occur, due to which it is a slow safety mechanism.

More recently, a new type of suicide gene based on the apoptotic activity of caspase 9 has arisen (Straathof, K.C., et al., 2005, Blood, 105(11): p. 4247-54). Caspase 9 is produced in cells as an inactive monomer that changes to a functional state once dimerized. In its natural state, dimerization is subject to the physiological state of the cell. However, in order to make it independent from this condition and place it under external control, the dimerization domain inherent to the gene, *Caspase Recruitment Domain* (CARD), has been substituted for a variant of the FK506 Binding Protein (FKBP12) containing the F36V mutation in the caspase 9-based suicide genes. This mutation causes the domain to become dimerized in the presence of any of the AP20187 synthetic molecules (also called CID or "chemical inducer of dimerization") or AP1903 but does not respond to the endogenous dimerizer, which makes it exclusively dependent on external actions. The addition of AP20187 induces the dimerization of the FKBP12-caspase 9 hybrid molecule, which activates the caspase 9 and triggers the apoptotic cascade that causes cell death within a few minutes. This inducible caspase 9 system (also called iCaspase or iCaspase9) was tested *in vitro* and *in vivo* with apparently better results than those of TK, but was also unable to completely eliminate the target cells (Kemper, K., et al., 2012, Apoptosis: an international journal on programmed cell death, 17(5): p. 528-37).

Some patent documents, such as WO2016113702 or US5631236, also include the application of the HSV-TK suicide gene as a genetic safety mechanism in cell therapy with stem cells or in solid tumors, respectively. Furthermore, patent documents such as EP3021853A1 or US2015366954 disclose the induction of selective apoptosis through the iCaspase9 inducible suicide gene.

Currently only the two HSV-TK/GCV and iCasp9/AP1903 have managed to reach the clinical testing stage to increase cell therapy safety in malign hematological processes.

When the two suicide strategies, iCaspase and TK, were compared directly in a single experiment both exhibited similar effectiveness, although the iCaspase acts faster (Marin, V., et al., 2012, Human gene therapy methods, 23(6): p. 376-86). However, the effectiveness of the iCaspase system is considerably reduced in cells with a low or intermediate level of expression of this transgene, which negatively impacts its functionality at a clinical level (Di Stasi A, Tey SK, Dotti G, et al., 2011, N Engl J Med., 365(18):1673-1683).

Other suicide genes described to date are the mutant human thymidylate kinase (mTMPK) enzyme, wherein the enzyme-expressing cells are susceptible to therapeutic doses of the Zidovudine (AZT) agent (Sato et al., 2007, Mol. Ther. 15, 962-970). The expression of the CD20 antigen was also proposed as a suicide strategy (Serafini et al., 2004, Hum. Gene Ther., 15:6, 3-76). The cells that express the gene that encodes it can be selectively destroyed by the administration of the therapeutic antibody rituximab. The main drawback of this strategy is the concomitant transitional depletion of the B-cell compartment of the patient. Indeed, there are currently no clinical results using this system. Another proposed suicide tool is the cytosine deaminase (CD) gene from *E. coli* (CD). CD can be activated by the 5-FC (5-fluorouracyl) compound. This strategy has been shown to have cytotoxic effects on breast cancer, glioma, colon cancer and other tumors (Brade et al., 2003, Cancer Gene Ther, 10, 294-301).

However, due to the limitations of individual suicide gene therapy, the gene therapy was also assayed with suicide gene combinations; however, the lack of a promoter with specific activity limits the application of this approach in tumors. An example of fusion gene construct is the CD/TK double suicide system expressed on the basis of an adenoviral vector in the presence of the surviving promoter. This double system has been shown to have an improved effect, inhibiting the growth of tumors when activated by GCV and 5-FC in comparison with the effect of these two suicide genes individually applied (Xian-Run Luo, et al., 2012, Asian Pacific Journal of Cancer Prevention, Vol 13, pp-781-784).

However, since clinical trials performed to date using suicide gene constructs have only been able to partially eliminate the construct-bearing cells, the need arises for an improved suicide gene system that will make it possible to selectively eliminate, with greater effectiveness and reliability, specific cell populations in the interior of an organism when their presence poses a health risk, minimizing the presence of target cells resistant to the treatment.

### DESCRIPTION OF THE INVENTION

The present invention provides a double and inducible suicide gene construct that combines the cell death-inducing capacity of the two suicide genes, HSV-TK/GCV and iCaspase9. Therefore, the effect of both suicide genes is complementary, providing a safety mechanism endowed with redundancy, making it more effective and reliable.

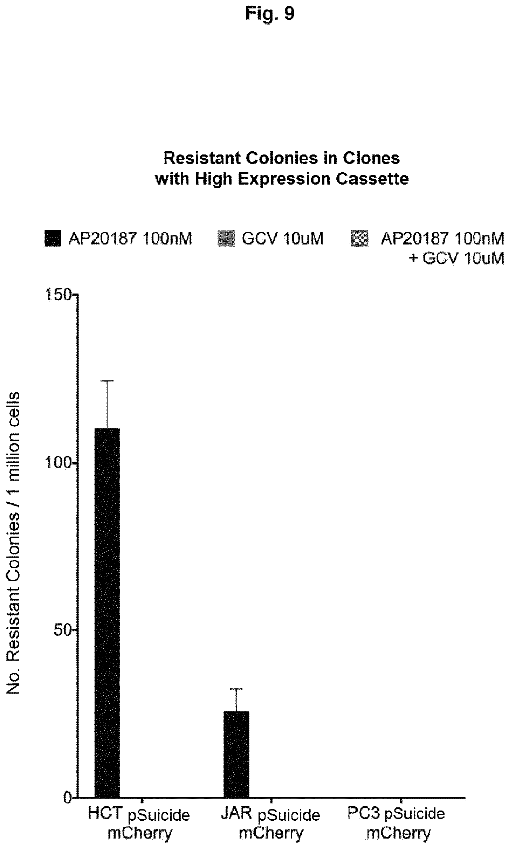

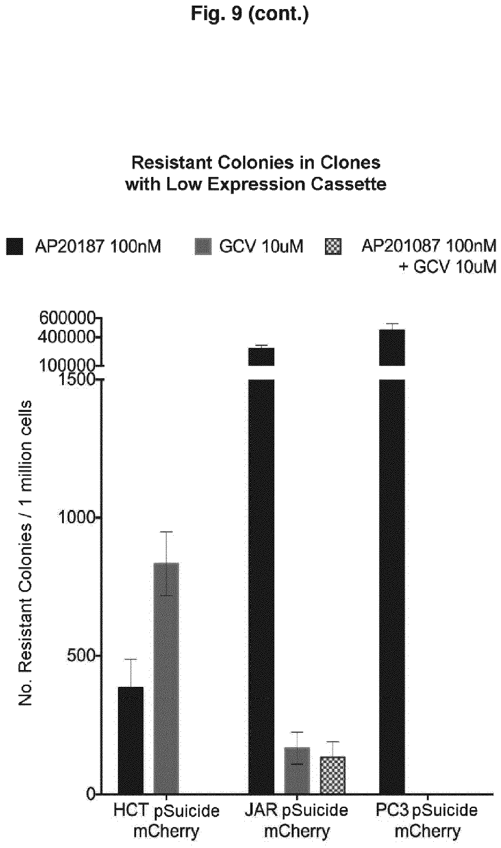

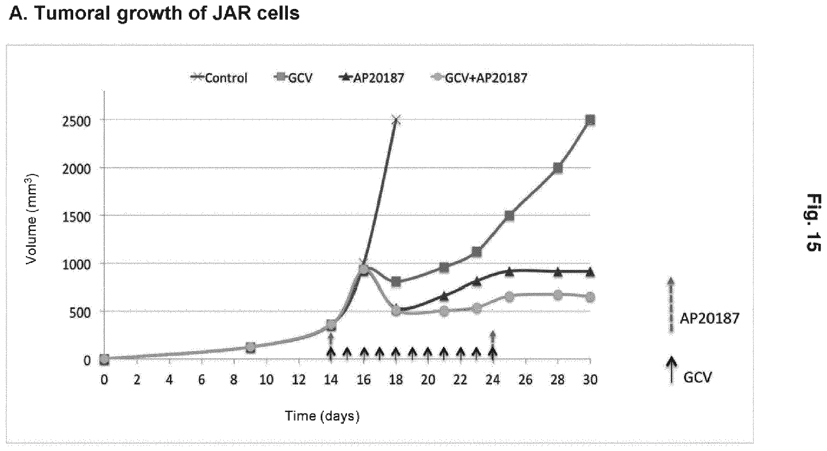

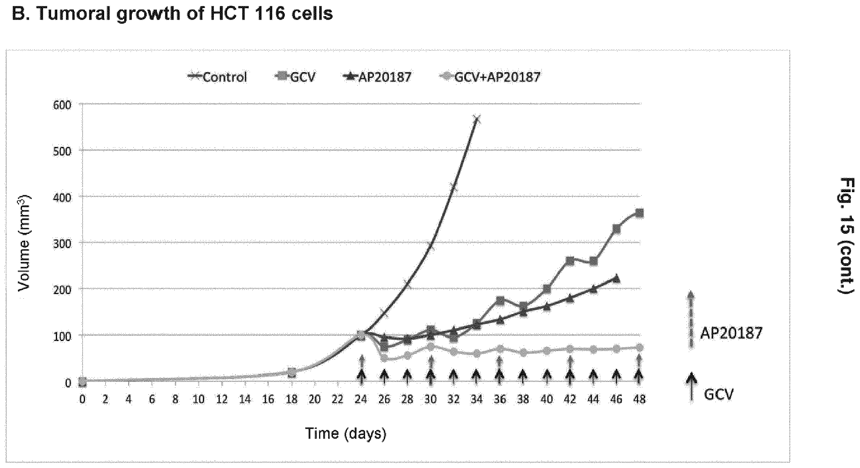

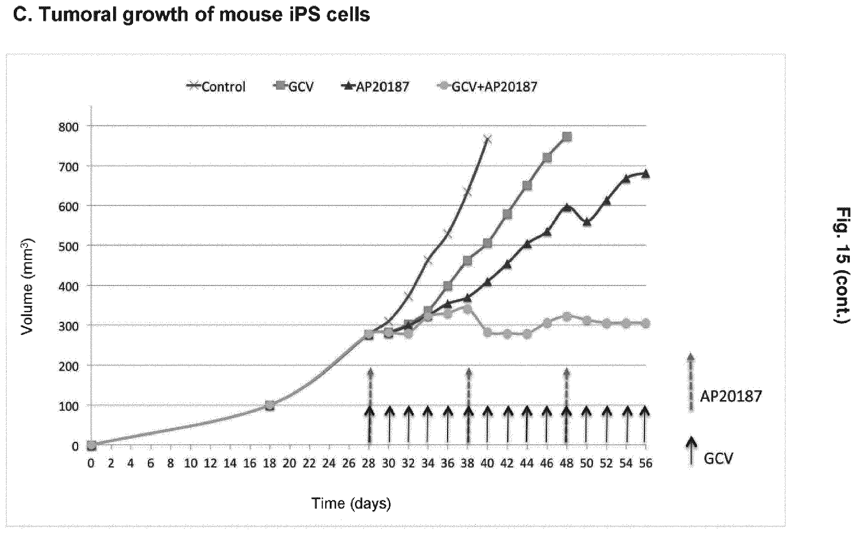

This double gene construct has also been shown to have, *in vivo,* an effect on cell death induction, when both genes are simultaneously activated with their corresponding inductors or effectors, clearly superior to each of these two suicide genes separately (see examples shown below). In particular, the number of cell colonies comprising the construct that were resistant to the combined treatment was lower than when the two treatments were administered separately (see Fig. 9), completely stopping and even slowing tumor growth (see Fig. 15).

Therefore, the suicide gene construct described herein is the most adequate for the clinical application of all those described to date. Therefore, the present invention represents a solution to the problem proposed, by providing an improved suicide gene system that selectively eliminates unwanted cell populations more effectively.

The immediate usefulness of this system lies in its use in transplanted cells which, as a result of having been handled *ex vivo,* may represent a risk after being transferred to the receptor organism. Additionally, the examples shown below demonstrate the effectiveness of this suicide gene construct both in human tumor cell lines and in transplanted human stem cells. Therefore, the gene construct described herein may be applied, but not limited to, cell or tissue transplants in regenerative medicine, including "Adoptive T-cell therapy" or adoptive T-lymphocyte therapy, and directly in endogenous tumor cells if the construct is introduced therein by means of specifically directed vectors. Another possibility would be its use to induce death "by contagion" in cancer cells due to their proximity to the suicide construct-bearing cells located in the vicinity of the tumor. In general, this construct is useful as a genetic safety mechanism under any circumstance in which it is necessary to eliminate a specific cell population in the interior of an organism at will, specifically and without need for physical intervention.

The polycistronic gene construct or suicide cassette of the invention comprises an encoding sequence for the iCaspase9 and an encoding sequence for the HSV-TK. This gene construct was also designed to minimize the risk of inactivation by the endogenous cell defense mechanisms and maximize the expression of the two suicide proteins. In particular, this construct has various features that make it optimal for guaranteeing the best possible operation of a suicide cassette. Firstly, the presence of two 2A elements enables expression of at least two proteins at the same level, preferably three when a marker gene is co-expressed, from a single, preferably constitutive eukaryotic promoter. The inclusion of said marker gene in the cassette makes it possible to detect and quantify the expression level of the construct. Furthermore, if one human promoter is included, such as EF1-α promoter, the possibility of silencing or reducing its activity by the cell defense mechanisms when the construct is applied to human cells is reduced. Additionally, the presence of a β-globin intron maximizes the transcription of the genes comprised in the cassette. Lastly, the coexistence in the cell of each of the two suicide proteins at similar levels makes it possible to compare its activity directly and without biases due to integration or expression effects.

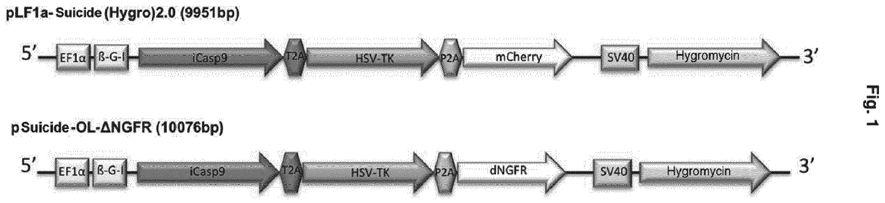

Fig. 1 shows a diagram of the preferred structure of the gene construct of the invention.

Due to the foregoing, a first aspect of the invention relates to a polycistronic gene construct (hereinafter, "gene construct of the invention" or "expression cassette of the invention") comprising:
a) a gene promoter, preferably of eukaryotic origin,
b) an intron, preferably that of beta-globin or beta-actin, more preferably that of beta-globin,
c) an encoding nucleotide sequence for the icaspase9 protein,
d) a first translation initiation or continuation element from the interior of the polycistronic messenger, preferably of the 2A type,
e) an encoding nucleotide sequence for the herpes simplex virus thymidine kinase protein,
f) a second translation initiation or continuation element from the interior of the polycistronic messenger, preferably of the 2A type, and
g) preferably at least one marker gene,
wherein more preferably elements (a) to (g) are disposed in the 5' to 3' sense in said gene construct.

The term "gene construct" or "expression cassette" relates to the DNA unit wherein a genetic material of interest can be introduced to be expressed as a protein or RNA in a cell. The elements comprised within the gene construct are in a reading frame or "operationally joined together", i.e. sequentially oriented therebetween in such a manner as to allow them to function in the intended manner, allowing the expression (i.e. the transcription and translation) of the encoding sequence(s) to take place in compatible conditions with the other elements or sequences present in the construct.

In the present invention, "polycistronic" is understood to be the tandem arrangement of various genes. Thus, a "polycistronic gene construct", such as that of the present invention, encodes for different multiple polypeptides.

As used herein, the term "gene promoter" refers to a DNA region or sequence, generally situated "upstream" of the starting point of the transcription, which is capable of initiating the transcription of a nucleotide sequence in a cell. This term includes, for example, but not limited to, constitutive or inducible/repressible promoters, as well as specific cell or tissue-type promoters or ubiquitous promoters. Preferably, the promoter to which the invention relates is constitutive. The origin of the selected promoter will depend on the cell in which the gene construct of the invention is intended to be expressed. Thus, promoters of eukaryotic, prokaryotic or viral origin may be used. Examples of prokaryotic promoters include, but not limited to, *E. coli* trp, recA, lacZ, lacI, tet, gal, trc, or tac gene promoters, or the B. *subtilis* α-amylase gene promoter. Examples of viral promoters are, but not limited to, the cytomegalovirus promoter, the SV40 promoter, of the Rous sarcoma virus LTR, of the murine leukemia virus LTR or of the herpes simplex virus thymidine kinase. Examples of eukaryotic promoters are, but not limited to, the human beta actin promoter, TRE, UAS, Ac5, of polyhedrin, CaMKllla, GAL1, 10, TEF1, GDS, ADH1, CaMV35S, H1, U6, B29, of CD14, of CD43, of CD45, of CD68, of desmin, of elastase-1, of endoglin, of fibronectin, of GFAP, of Flt-1, EF1alfa, PGK, CAG or UBC.

In a preferred embodiment, the promoter comprised in the gene construct of the invention is a gene promoter of eukaryotic origin, more preferably human, even more preferably it is a constitutive promoter of human origin. Some examples of promoters of this type that could be used in the construct are, but not limited to, the PGK1 (phosphoglycerate kinase) promoter, the ubiquitin C (UBC) promoter, the EF1alpha and β-actin (ACTB) promoter. In a particular embodiment, the promoter is EF1alpha (Human Elongation Factor 1 alpha), more preferably wherein said EF1alpha promoter is that shown in the sequence SEQ ID NO: 6.

Due to the degeneration of the genetic code, wherein various nucleotide triplets give rise to the same amino acid, there are various nucleotide sequences that give rise to a same amino acid sequence. The terms "nucleotide sequence", "sequence of nucleotides", "nucleic acid", "oligonucleotide" and "polynucleotide" are used herein interchangeably and relate to a polymer form of nucleotides of any length that may or may not be chemically or biochemically modified. Therefore, they refer to any polyribonucleotide or polydeoxyribonucleotide, both single-stranded and doublestranded. The encoding nucleotide sequences of the present invention can be artificially obtained by means of cloning methods and conventional selection, or sequencing. The encoding nucleotide sequences of the present invention may comprise other elements such as, for example, but not limited to, introns, noncoding sequences at terminal ends 5' or 3', ribosome binding sites or stabilizing sequences. These polynucleotides may also include encoding sequences for additional amino acids that may be useful, for example, but not limited to, increasing the stability of the peptide generated therefrom or enable simplified purification thereof.

In another preferred embodiment of the present invention, the "encoding nucleotide sequence for the icaspase9 protein" is SEQ ID NO: 1 or any sequence arising therefrom by codon optimization. In another preferred embodiment of the present invention, the "encoding nucleotide sequence for the herpes simplex virus thymidine kinase protein" is SEQ ID NO: 2 or any sequence arising therefrom by codon optimization.

The "beta-globin intron" is, preferably, that described in SEQ ID NO: 3.

The "icaspase9", "inducible caspase 9", "iCasp9" or "iC9" protein is the exogenously inducible caspase 9 protein, i.e. whose activation or dimerized state is independent from intracellular factors and depends solely on external action by administering a specific dimerizing or effector molecule. This icaspase9 is the polypeptide described in literature, for example, but not limited to, in Di Stasi, et al., 2011, N. Engl. J. Med. 365, 1673-1683. Said icaspase9 comprises a substitution in its dimerization domain, *Caspase Recruitment Domain* (CARD, GenBank NM001229), for a variant of the FK506 Binding Protein containing the F36V mutation (FKBP12, GenBank AH002818). That is, icaspase9 comprises the FKBP12-F36V domain bound, via a linker, to Δcaspase 9, wherein Δcaspase 9 is the caspase 9 protein without its CARD physiological dimerization domain. In another preferred embodiment, the amino acid sequence of icaspase9 is SEQ ID NO: 4.

The "herpes simplex virus thymidine kinase" or "HSV-TK" protein is that described in, for example, but not limited to, Ciceri, F., et al., 2007, Blood 109, 4698-4707. In another preferred embodiment, the amino acid sequence of HSV-TK is SEQ ID NO: 5.

The "translation initiation or continuation element" to which reference is made herein can be selected, for example, but not limited to, from a 2A-type element or an IRES element. Preferably, the first translation initiation or continuation element is of the 2A type and the second translation initiation or continuation element is IRES. More preferably, both the first and second translation initiation or continuation element is of the 2A type.

The "2A element" to which the present invention relates is an encoding nucleotide sequence for a 2A peptide, which is an oligopeptide between 19 and 22 amino acids in length that is split off from the finally expressed polypeptide with which it is co-expressed. This nucleotide sequence can be obtained, for example, from a Thosea asigna (T2A) virus or foot-and-mouth disease virus (FMDV). This 2A peptide is usually found between two proteins in viruses of the picornavirus family. It can be obtained, for example, but not limited to, from porcine teschovirus-1 (P2A), from equine rhinitis A (E2A) virus, from cytoplasmic polyhedrosis (BmCPV 2A), from flacherie virus (BmIFV 2A), from foot-and-mouth disease virus, from Avisivirus A virus, from duck hepatitis A virus, from encephalomyocarditis virus, from Cosavirus A virus, from Seneca Valley virus, from Hunnivirus A virus, from Kunsagivirus A virus, from Mischivirus A virus, from Mosavirus A2 virus or from Pasivirus A1. It can also be obtained from other virus families such as, for example, but not limited to, the Iflaviridae, Tetraviridae, Dicistroviridae and Reoviridae families. This 2A element enables the expression of two or more functional proteins from a single gene construct. In the gene construct of the invention, the first and second 2A element can be identical to or different from each other, preferably different. In another preferred embodiment, the first 2A element comprised in the gene construct of the invention is T2A and the second 2A element is P2A. More preferably, the T2A element is the peptide with the sequence SEQ ID NO: 7 and is encoded by the nucleotide sequence of SEQ ID NO: 8. Even more preferably, the P2A element is the peptide with SEQ ID NO: 9 and is encoded by the nucleotide sequence of SEQ ID NO: 10.

The "marker gene" to which the present invention relates may be any gene whose expression product can be identified, detected and/or quantified due to the fact that it provides an identifiable change in the cell that expresses it. Thus, the presence and expression of the marker gene in the gene construct of the invention will make it possible to easily identify the cells that comprise said construct. Said marker gene may be, for example, but not limited to, a gene that confers resistance to metals or to medicaments, preferably to antibiotics, such as for example genes conferring resistance to neomycin, puromycin, hygromycin, DHFR, GPT, zeocin, histidinol, streptomycin, ampicillin or similar. Inducible or non-inducible reporter genes, such as GFP, EGFP, mCherry, luciferase, IRV3, or any other fluorescent or chemo or bioluminiscent protein, beta-galactosidase, chloramphenicol acetyltransferase (CAT), horseradish peroxidase (HRP) or similar. Encoding marker genes can also be used for epitopes or immunogenic molecules, such as for example, but not limited to, FLAG, HA, His, Myc, V5, Xpress, Thrombin, DAD, S Tag, C Protein or for truncated receptors expressed on the cell surface, such as CD34, CD19, ΔNGFR or ΔNGFR. The selection of the marker gene is not relevant provided that it can be simultaneously expressed with encoding nucleotide sequences indicated in elements (c) and (e) of the gene construct of the invention. Preferably, the marker gene to which the present invention relates is the encoding gene for the truncated membrane receptor ΔNGFR, whose clinical use is accepted and proven. More preferably, the encoding gene for the truncated membrane receptor ΔNGFR is SEQ ID NO: 11 and the truncated membrane receptor ΔNGFR that it encodes is SEQ ID NO: 12.

The gene construct of the invention can be introduced in a cloning or expression vector to enable its replication and expression in the interior of a cell. Preferably, said vector is an appropriate vector for expressing the polypeptides encoded by the gene construct of the invention. Accordingly, another aspect of the present invention relates to an expression vector, (hereinafter, "gene construct of the invention"), comprising the gene construct of the invention.

The term "expression vector" or "cloning vector", as used in the present invention, relates to a nucleic acid construct, preferably DNA, wherein the gene construct of the invention can be integrated without said vector losing its replication capacity. The expression vector is designed to direct the transformation or transduction of said gene construct or an encoding nucleotide sequence for one or more genes of interest, in the interior of a target cell and its subsequent replication and expression. In addition to the gene construct of the invention, the vector comprises other genetic elements sequentially oriented (operationally bound) in such a manner that the gene construct of the invention may be transcribed and translated in the cell. Said genetic elements may be, but not limited to, a promoter, a terminator, a leader sequence, a transcription initiation site, one or more replication origins, an untranslated regulatory region 3' and/or 5', a potentiator, a polyadenylation signal or any other control sequence. The expression vector may also comprise sequences that direct the expression of the gene construct of the invention comprised therein in a specific cell type, in a specific cell sublocation or in a specific tissue. Preferably, said vector comprises sequences that direct the expression of the gene construct of the invention specifically in tumor cells.

The expression vector of the invention can be selected, but not limited to, from a plasmid, phage, cosmid, phagemid, autonomously replicating sequence (ARS), yeast artificial chromosome (YAC), bacterial artificial chromosome (BAC), human artificial chromosome (HAC) or viral vectors such as, but not limited to, lentivirus, baculovirus, adenovirus, retrovirus, adeno-associated viral vector (AAV) or any other type of DNA molecule capable of replicating in the interior of a prokaryotic or eukaryotic cell, preferably eukaryotic. Preferably, said vectors are the pLF1a-Suicide(Hygro)2.0 and pSuicide-OL-ΔNGFR vectors. In another preferred embodiment, the expression vector is a viral vector.

The expression vector of the invention may be a self-replicating vector, whose replication is independent from the genome of the cell in which it is introduced, or may be a vector which becomes integrated in the genome of the target cell once transfected and replicates therewith. Thus, the vector may comprise sequences that facilitate its insertion in a specific location within the genome of the host cell.

Another aspect of the invention relates to an isolated *(ex vivo)* cell or cell population that comprises (and preferably expresses) the gene construct of the invention or the expression vector of the invention. Hereinafter, this cell or cell population will also be referred to as "cell or cell population of the invention". This aspect of the invention also applies to the progeny of this cell or cell population of the invention.

The cell or cell population of the invention was transduced, transfected, transformed or similar with the gene construct or expression vector of the invention using any genetic engineering technique from those known in the art for such purpose. Said techniques may be, for example, but not limited to, injection or microinjection, ex *vivo* transformation, electroporation, precipitation with calcium phosphate, DEAE-dextrane followed by polyethylene glycol, sonication, biolistics, retroviral infection, liposome-mediated transfection (lipofection) or via receptor, or other techniques widely known in the state of the art.

The terms "transfection", "transduction" and "transformation" are used herein interchangeably to define the methods whereby the genetic material of a cell has been altered, preferably increased, through the introduction of the gene construct or expression vector of the invention using genetic engineering techniques.

The cell or cell population of the invention may be of allogeneic, xenogeneic or autologous origin, although it preferably has an autologous origin.

The term "allogeneic" refers to a cell of another subject or individual of the same species as the subject or individual to which said cell will be administered subsequent to the *ex vivo* manipulation thereof.

The term "xenogeneic" refers to a cell of another subject or individual of a different species to the subject or individual to which said cell will be administered subsequent to the *ex vivo* manipulation of said cell.

The term "autologous" refers to a cell of the same subject or individual as the subject or individual to which said cell will be administered subsequent to the *ex vivo* manipulation thereof.

In another preferred embodiment, the cell or cell population of the invention is eukaryotic. Said cell may be of human or of non-human animal origin, such as for example non-human mammals such as, but not limited to, mouse, rat, monkey, pig, rabbit, dog or cat. More preferably, the cell or cell population of the invention is human.

In a particular embodiment, the cell or cell population of the invention is human and autologous.

In another preferred embodiment, the cell or cell population of the invention is a cell or cell population of the immune system, more preferably a T-lymphocyte or population of T-lymphocytes, wherein the term "T-lymphocyte" also includes T-lymphocytes modified for chimeric antigen receptor-surface expression (also called CART cells).

Examples of "immune system cells" are, but not limited to, polymorphonuclear cells, monocytes, neutrophils, macrophages, basophils, mastocytes, eosinophils, dendritic cells, microglia, Kupffer cells, follicular dendritic cells, NK cells or lymphocytes.

A "T-cell" or "T-lymphocyte" is an immune system cell belonging to the group of white blood cells generally involved in cell immunity and whose maturity takes place in the thymus. It has cell surface proteins known as "T-cell receptors". These cells are activated by the presence of an antigenic determinant, cytokines and/or lymphokines. The term "T-lymphocyte" includes the helper T-lymphocytes or CD4+, the cytotoxic T-lymphocytes or CD8+, the memory T-lymphocytes, the regulatory T-lymphocytes and gamma-delta T-lymphocytes.

In another preferred embodiment, the cell or cell population of the invention is tumoral or a stem cell or stem cell population or differentiated stem cell-derived cells.

The "tumor cell" to which the present invention relates is any cell from a benign or malign tumor, preferably malign, more preferably a cancer cell. Said cell is, more preferably, from a prostate carcinoma, from a choriocarcinoma or colon carcinoma.

The "stem cell" to which the present invention relates may be, but not limited to, totipotent, pluripotent, induced pluripotent (iPSC), multipotent, unipotent or progenitor, germ or resident stem cell in an adult tissue, for example a hematopoietic stem cell (HSC), adipose stem cell or mesenchymal stem cell. Also, said cell may be an adult, embryonic or non-human, or human embryonic cell obtained by means of a method that does not compromise the viability of the embryo. The stem cell to which the present invention relates may be from, for example, but not limited to, umbilical cord, amniotic fluid, bone marrow, peripheral blood, adipose tissue, muscle, cornea or dental pulp or from any niche stem cell present in the tissues of an individual. In a more preferred embodiment, the stem cell of the invention is a neural progenitor cell, a hematopoietic progenitor cell, an adipose stem cell, a mesenchymal cell or iPSC, even more preferably an iPSC.

An "induced pluripotent stem cell" or "iPSC" is an adult or differentiated cell which has been reprogrammed or induced to dedifferentiate itself using genetic (due to the expression of certain genes), biological (due to the treatment with, for example, virus or retrovirus) and/or chemical (with small molecules, peptides and similar) techniques, so as to activate its pluripotent capacity. iPSCs are therefore capable of differentiation in most or all cell types, as in the case of embryonic stem cells.

A "hematopoietic progenitor cell" is a cell capable of differentiating itself from the mature blood cell types. These cells may be isolated, for example, from bone marrow, umbilical cord or peripheral blood.

A "differentiated stem cell-derived cell" is a stem cell which has been artificially (i.e. by human intervention) induced to differentiate itself, *in vivo* or *in vitro,* in a cell type of interest using genetic, biological and/or chemical techniques, for example which has been subject to culture conditions that make it possible to acquire the differentiated state of interest.

Another aspect of the invention relates to a combined composition or preparation, (hereinafter, "composition of the invention"), comprising:
i) the gene construct of the invention, the expression vector of the invention or the cell or cell population of the invention,
ii) a substrate of the herpes simplex virus thymidine kinase protein, and
iii) an icaspase9 protein activator.

Alternatively, this composition of the invention may comprise elements (ii) and (iii) shown above and not comprise element (i), in which case said composition would be administered to an organism or subject in which the gene construct, vector or cell or cell population of the invention has been previously introduced.

The term "combined preparation", also called "juxtaposition", means that the components of the composition do not need to be present as a bond in order to be available for the separate or sequential application thereof. Therefore, the expression "juxtaposed" implies that it is not necessarily a true combination, in view of the physical separation of the components.

Preferably, the "substrate of the herpes simplex virus thymidine kinase protein" to which reference is made throughout the description and claims is selected from the list consisting of: Ganciclovir, Aciclovir, AZT, Stavudine, Idoxuridine or FIAU (1-(2-deoxy-2-fluoro-1-D-arabinofuranoside)-5-iodouracil). More preferably, said substrate is Ganciclovir (CAS Number: 82410-32-0).

Preferably, the "icaspase9 protein activator" to which reference is made throughout the description and claims is AP20187 or an analog thereof, such as for example AP1510, or AP1903 (CAS Number: 195514-63-7). More preferably, said activator is AP20187 (CAS Number: 195514-80-8).

In another preferred embodiment, the composition of the invention is a pharmaceutical composition, wherein more preferably said pharmaceutical composition also comprises a pharmaceutically acceptable carrier, an excipient and/or other active ingredient, even more preferably a pharmaceutically acceptable carrier.

The term "excipient" refers to a substance which aids the absorption of the elements of the composition of the invention, preferably of elements (ii) and (iii), stabilizes said elements, activates or aids the preparation of the composition in the sense of giving it consistency or providing flavors that make it more pleasant. Therefore, the excipients may have the function of holding the ingredients together such as, for example, in the case of starches, sugars or celluloses, a sweetening function, a coloring function, a composition protection function, such as for example, to isolate it from the air and/or humidity, the function of filling a tablet, capsule or any other form of presentation, such as for example, in the case of dibasic calcium phosphate; the disintegrating function to facilitate the dissolution of the components, preferably of elements (ii) and (iii), and their absorption in the intestine, without excluding other types of excipients not mentioned herein.

The "carrier", like the excipient, is a substance used in the composition to dilute any of the components of the present invention comprised therein up to a certain volume or weight. The pharmacologically acceptable carrier is an inert substance or with action identical to any of the elements comprised in the composition. The function of the carrier is to facilitate the inclusion of other elements, allow better dosing and administration or give the composition consistency and shape. The carrier may be a diluent, coadjuvant or vector with which the pharmaceutical composition of the invention must be administered. When the form of presentation is liquid, the pharmacologically acceptable carrier is the diluent. The carrier may be a liquid, such as water, solvent, oil or surfactant, including those of petroleum, animal, vegetable or synthetic origin.

The composition of the invention, preferably in terms of elements (ii) and (iii) (activators and substrates), can be administered jointly with a sustained release carrier. The term "sustained release carrier" refers to a vehiculization system that provides the gradual release of the compounds comprised in the composition during a period of time and preferably, although not necessarily, with relatively constant release levels over said period of time. Examples of sustained release carriers or systems include, but are not limited to, liposomes, mixed liposomes, oleosomes, niosomes, etosomes, millicapsules, microcapsules, nanocapsules, sponges, cyclodextrins, vesicles, micelles, mixed surfactant micelles, mixed phospholipid surfactant micelles, millispheres, microspheres, nanospheres, lipospheres, microemulsions, nanoemulsions, miniparticles, milliparticles, microparticles, nanoparticles, solid lipid nanoparticles or nanostructured lipid media.

The term "active ingredient", "active substance", "pharmaceutically active substance", "active ingredient" or "pharmaceutically active ingredient" refers to any component that potentially provides a pharmacological activity or other different effect on the diagnosis, cure, mitigation, treatment or prevention of a disease, or that affects the structure or function of the human body or that of other animals. The term includes those components that promote a chemical change in the manufacture of the medicament and are present therein in an envisaged modified form that provides the specific activity or effect.

The composition of the invention, preferably in terms of elements (ii) and (iii), can be presented in the form of a solid, liquid, semi-solid (for example, gel) or granulated formulation. Examples of this type of formulations are, but not limited to, creams, multiple emulsions, anhydrous compositions, aqueous dispersions, oils, milks, balsams, foams, lotions, gels, cream gels, hydroalcoholic solutions, hydroglycolic solutions, hydrogels, liniments, serums, ointments, mousses, pomades, powders, bars, sticks, sprays, aerosols, capsules, gelatin capsules, soft capsules, hard capsules, pills, tablets, sugar-coated tablets, granulated forms, solutions, suspensions, emulsions, syrups, jellies, gelatin, liposomes or nanospheres or any other conventional sustained release format.

The composition of the present invention, preferably in terms of elements (ii) and (iii), can be administered to an animal, including a mammal and, therefore, to humans, in a variety of ways including, but not limited to, oral, parenteral, intraperitoneal, intravenous, intradermal, epidural, intraspinal, intrastromal, intraarticular, intrasinovial, intratecal, intralesional, intraarterial, intracardiac, intramuscular, intranasal, intracraneal, subcutaneous, intraorbital, intracapsular, topic, by means of transdermal patches, percutaneous, nasal spray, surgical implant, internal surgical paint, infusion pump or catheter.

The composition of the invention comprises elements (i) to (iii) in a therapeutically effective amount, understanding "therapeutically effective amount" to be the amount of gene construct, expression vector or cell or cell population of the invention, of HSV-TK protein substrate and of icaspase9 protein activator, that produces the desired effect, i.e. directed and specific cell death. The dose for obtaining a therapeutically effective amount will depend on a variety of factors, such as for example, age, weight, sex, pathological condition or tolerance of the individual to which the composition of the invention will be administered. The persons skilled in the art will know how to adjust said dose in accordance with these and other parameters.

Additionally, the composition of the invention may be used both alone and in combination with other compositions or medicaments used in the treatment and/or prevention of pathologies, diseases, syndromes, disorders, pathological or physiological conditions with the unwanted presence and/or proliferation of cells, preferably for the treatment and/or prevention of tumors, of graft-versus-host disease or transplant rejection. Thus, the composition of the invention may be used jointly with other active ingredients, comprised therein or not, or therapies in the manner of a combined therapy.

Preferably, the composition of the invention is formulated for the simultaneous or sequential administration of elements (ii) and (iii) comprised therein, more preferably for simultaneous administration.

Another aspect of the invention relates to the gene construct of the invention, to the expression vector of the invention, to the cell or cell population of the invention or to the composition of the invention, for use as a medicament. Alternatively, this aspect of the invention relates to the use of the gene construct of the invention, the expression vector of the invention, the cell or cell population of the invention or the composition of the invention, for the manufacture of a medicament.

In a preferred embodiment, and particularly when the medicament to which the present invention relates comprises the cell or cell population of the invention, said medicament is a cell therapy medicament, preferably for use in transplants.

In another preferred embodiment, and particularly when the medicament to which the present invention relates comprises the gene construct or the expression vector of the invention, the medicament of the invention is a gene therapy medicament.

The term "cell therapy" or "cytotherapy" refers to the therapy in which the patient is administered living cell material or cells, in the context of the present invention the cell or cell population of the invention.

The term "gene therapy" refers to the therapy in which genetic material is introduced, in the context of the present invention the gene construct or the expression vector of the invention, in the cells of the subject to be treated.

The terms "patient", "organism", "individual" or "subject" are used herein interchangeably and include, but not limited to, an animal, preferably a mammal, such as for example a human, non-human primate (for example, monkey), rodent (for example, mouse, rat, hamster, rabbit), pig, cow, sheep, goat, horse, etc. The subject to which the present invention relates may also be a bird or a fish. In a more preferred embodiment, said subject is a human.

The term "medicament" refers to any substance used to prevent, alleviate, treat or cure pathologies, diseases, syndromes, disorders, pathological or physiological conditions with the unwanted presence and/or proliferation of cells; preferably tumors, of graft-versus-host disease or transplant rejection.

The medicament to which the present invention relates may be for human or veterinary use. The "medicament for human use" is any substance or combination of substances with properties for treating or preventing diseases in humans or that can be used in humans or administered to humans for the purpose of restoring, correcting or modifying physiological functions exerting a pharmacological, immune or metabolic effect, or establishing a medical diagnosis. The "medicament for veterinary use" is any substance or combination of substances having curative or preventive properties with respect to animal diseases or that can be administered to the animal for the purpose of restoring, correcting or modifying its physiological functions exerting a pharmacological, immune or metabolic effect, or establishing a veterinarian diagnosis.

Another aspect of the invention relates to the gene construct of the invention, to the expression vector of the invention, to the cell or cell population of the invention or to the composition of the invention for use in the induction of directed and selective or specific cell death or for use in the elimination of unwanted target cells. Alternatively, this aspect of the invention relates to the use of the gene construct of the invention, of the expression vector of the invention, of the cell or cell population of the invention or of the composition of the invention to induce directed and selective or specific cell death or to eliminate unwanted target cells.

Another aspect of the invention relates to the gene construct of the invention, to the expression vector of the invention, to the cell or cell population of the invention or to the composition of the invention, for use in the treatment and/or prevention of pathologies, diseases, syndromes, disorders, pathological or physiological conditions in which an unwanted presence and/or proliferation of certain cells is involved. Alternatively, this aspect of the invention relates to the use of the gene construct of the invention, the expression vector of the invention, the cell or cell population of the invention or the composition of the invention, for the manufacture of a medicament for the treatment and/or prevention of pathologies, diseases, syndromes, disorders, pathological or physiological conditions in which an unwanted presence and/or proliferation of certain cells is involved.

Another aspect of the invention relates to the gene construct of the invention, to the expression vector of the invention, to the cell or cell population of the invention or to the composition of the invention, for use in the treatment and/or prevention of tumors, preferably cancer, graft-versus-host disease or transplant rejection. Alternatively, this aspect of the invention relates to the use of the gene construct of the invention, of the expression vector of the invention, of the cell or cell population of the invention or of the composition of the invention, for the manufacture of a medicament for the treatment and/or prevention of tumors, preferably cancer, graft-versus-host disease or transplant rejection.

In another preferred embodiment of the present invention, the substrate of the herpes simplex virus thymidine kinase protein and the activator of the icaspase9 protein comprised in the composition of the invention are administered simultaneously.

The term "treatment" refers to fighting the effects of the disease or pathological condition of interest in a subject (preferably mammal, and more preferably human) that includes:
(i) inhibiting the disease or pathological condition, i.e. stop its development;
(ii) alleviating the disease or pathological condition, i.e. cause the partial or total remittance of the disease or pathological condition or its symptoms;
(iii) stabilizing the disease or pathological condition.

The term "prevention" consists of avoiding the appearance of the disease or pathological condition, i.e. prevent the disease or pathological condition from appearing in a subject (preferably mammal, and more preferably human), in particular, when said subject has a predisposition for the pathological condition but has not yet been diagnosed with it.

The term "tumor", as used herein, refers to any type of neoplasm, i.e. it is not simply limited to malign neoplasms but rather also comprises benign neoplasms, dysplasias, hyperplasias or leukoplasias. Thus, the term "tumor" includes any neoplasic disease wherein cells, with abnormal morphology, exhibit uncontrolled growth, division or proliferation, generating a cell mass called a tumor, whether malign or benign.

The term "cancer" refers to a malign neoplasm wherein the organism comprises an excessive number of malign cells (cancer or cancerous cells), with uncontrolled treatment and division beyond normal limits, which in advanced stages leads to the invasion of the surrounding tissue and, lastly, to metastasis. It comprises any disease of an organ or tissue, preferably in a mammal, more preferably in a human, characterized by a poorly controlled or uncontrolled multiplication of normal or abnormal cells in said tissue, and its effect on the body as a whole. Cells and tissues are cancerous when they grow and replicate faster than normal, moving or dispersing in the surrounding healthy tissue or in any other body tissue, which is known as metastasis, adopt abnormal shapes and sizes, exhibit changes in their nucleocytoplasmic ratio, nuclear polychromasia, and finally ceases. Cancerous cells and tissues can affect the body as a whole, causing paraneoplasic syndromes, or may occur in a vital organ or tissue, interrupting or adversely affecting its normal function, with potentially fatal results. In general, cancer is said to be in one of three growth stages: early or localized, when the tumor is confined to the tissue of origin, or to its primary location; direct extension, when the cancer cells of the tumor have invaded the adjacent tissue or has extended only to the regional lymph nodules; or metastasis, when the cancerous cells have migrated to distant parts of the body from the primary location, via the circulatory or lymphatic system, and has become established in secondary locations. Cancer in any of these stages is comprised within the scope of the present invention.

Examples of "tumors" are, but not limited to, carcinoma, sarcoma, carcinosarcoma, adenocarcinoma, blastoma, hematopoietic tumors, lymphoid lineage tumors such as acute lymphocytic leukemia, acute lymphoblastic leukemia, lymphomas such as B- or T-cell lymphoma or Non-Hodgkin lymphoma; myeloid lineage tumors such as myeloma; nerve tissue tumors including the brain, astrocytoma, neuroblastoma, glioblastoma or glioma; skin tumors such as, for example, melanoma, seminoma, teratocarcinoma, keratoacanthoma, Kaposi's sarcoma; epithelial cell cancer or squamous cell carcinoma; fibrosarcoma, rhabdomyosarcoma, breast, prostate, bladder, colon and rectum, lung, kidney (renal cells), stomach, thyroid, esophagus, liver, pancreas, bone (osteosarcoma), thymus, endometrial or uterine, uterus neck or cervical carcinoma or cervical, ovarian, vaginal, vulvar, head and neck, salivary gland, urinary tract cancer, or any other solid tumor.

Preferably, the tumor to which the present invention relates is prostate carcinoma, choriocarcinoma or colon carcinoma.

In general, the medicament to which the present invention relates is useful in any situation in which a certain cell population must be eliminated, in a controlled and specific manner, in the interior of an organism due to causing adverse effects. That is, said medicament is useful for causing or inducing cell death only in those specific cells comprising the gene construct or expression vector of the invention which, in the interior of an organism, preferably in the interior of a tissue, exhibit uncontrolled or abnormal growth and/or whose presence triggers adverse effects on the individual's health (such as for example, an unwanted immune reaction or the formation of a tumor).

"Graft-versus-host disease" or "GvHD" refers to the clinical complication associated with bone marrow transplant, preferably allogeneic and at times also associated with non-irradiated blood transfusions in immunocompromised patients. This disease may occur when the functional cells of the immune system of the transplanted bone marrow recognize the receptor as foreign and trigger an immune response. This disease may be acute or chronic. The acute form can normally be observed in the first 100 days after the transplant or infusion and may affect the liver, skin, mucosa, hematopoietic system, bone marrow, thymus or similar, lungs and gastrointestinal tract. The chronic form normally begins 100 days or more after the transplant or transfusion and can attack the same organs as the acute form but can also affect the connective tissue and exocrine glands.

The term "transplant rejection" relates to the process wherein the immune system of a transplant receptor attacks the transplanted organ, cells or tissue. It includes both acute rejection (which can occur anytime from the first week after the transplant to up to 3 months later), hyperacute rejection (which occurs a few minutes after the transplant) and chronic rejection (which can occur for many years and the constant immune response of the receptor organism to the transplant slowly damages the transplanted tissues or organs).

Furthermore, the cell or cell population of the invention can be directly administered as a cell therapy medicament as part or all of a transplant or subsequent thereto; or as a cell therapy medicament to cause the death by contagion of unwanted cells, such as for example in tumor cells, which are located in the vicinity of said cell or cell population of the invention once transferred to the organism.

Thus, for example, prior to a transplant, the cells to be transplanted can be transduced or transfected with the gene construct or with the expression vector of the invention so that, once administered or transplanted to the receptor organism and in the event that they represent a problem, for example, immune rejection, they are susceptible to being eliminated through the administration, preferably simultaneous, of the HSV-TK substrate and of the icaspase9 protein activator. Alternatively, the cell or cell population of the invention can be administered in the vicinity of a transplant that is causing immune rejection or of a tumor so that they cause death by contagion in the transplanted cells or tumor cells once the corresponding activators and substrates have been administered.

Furthermore, the medicament to which the invention relates can be applied to T-cell therapy. In this case the T-cells, prior to being administered to the organism, are transduced or transfected with the gene construct or with the expression vector of the invention so that, once administered to the receptor organism and in the event that they represent a problem, for example, immune rejection, they are susceptible to being eliminated through the administration, preferably simultaneous, of the HSV-TK substrate and of the icaspase9 protein activator.

"T-cell therapy" or "adoptive T-cell therapy" refers to the *ex vivo* expansion and selection of T-lymphocytes that will be subsequently administered to a subject for therapeutic purposes. This therapy has been widely used in allogeneic hematopoietic stem cells transplantation (HSCT), wherein the infusion of the donor's lymphocytes gives rise to immune rejection. Also used, but not limited to, in the treatment of viral infections such as cytomegalovirus, Epstein Barr infection-derived lymphomas and other types of cancer, including carcinomas. The term "T-cell therapy", as used in the present invention, also includes therapy with T-lymphocytes that have been genetically manipulated to express chimeric antigen receptors (known as "CART *cell therapy"*)*.*

Another aspect of the invention relates to the use of the gene construct of the invention or of the expression vector of the invention to generate or obtain, *in vitro,* useful cells for a transplant. In this regard, another aspect of the invention relates to an *in vitro* method for obtaining, generating or preparing (therapeutic) cells useful for a transplant (hereinafter, "method of the invention"), comprising:
a) transfecting or transducing the cells to be transplanted with the gene construct of the invention or with the expression vector of the invention, and
b) optionally cultivating said transfected or transduced cells to obtain a sufficient number for a transplant.

In a preferred embodiment of this aspect of the invention, the cells are eukaryotic, more preferably human, even more preferably autologous. In another preferred embodiment, the cells mentioned in this method of the invention are T-lymphocytes or stem cells or differentiated cells derived from stem cells.

These cells to which the method of invention relates may be subsequently transplanted not only in situations requiring cell therapy for tissue regeneration or to reinforce the immune system, but also for the treatment of cancer if administered directly to the tumor or in the vicinity thereof.

The terms *"ex vivo"* or *"in vitro"* refer to outside of the human or animal body.

Another aspect of the invention relates to a method for transplanting cells to an individual, wherein said method comprises:
a) transfecting or transducing the cells to be transplanted with the gene construct of the invention or with the expression vector of the invention,
b) optionally culturing said transfected or transduced stem cells to obtain a sufficient number for a transplant,
c) transplanting or administering the cells obtained after step (a) or (b) to the receptor organism, and
d) optionally administering a substrate of the herpes simplex virus thymidine kinase protein and an icaspase9 protein activator simultaneously to the receptor organism
when seeking the elimination or death of the cells transplanted in step (c).

In a preferred embodiment of this aspect of the invention, the cells are eukaryotic, more preferably human, even more preferably autologous. In another preferred embodiment, the cells mentioned in this method of the invention are T-lymphocytes or stem cells or differentiated cells derived from stem cells.

The expression "transplant" or "administer" cells to a receptor organism makes reference to the introduction of said cells in the subject to be treated by means of any known technique that serves such purpose, whether by injection, infusion or similar.

Another aspect of the invention relates to the use of the gene construct of the invention, of the expression vector of the invention or of the composition of the invention to induce cell death in cultured cells, preferably eukaryotic cells, more preferably human.

Another aspect of the invention relates to an *in vitro* method for inducing cell death in cultured cells, preferably eukaryotic, more preferably human, comprising:
a) transfecting or transducing the cells with the gene construct of the invention or with the expression vector of the invention,
b) culturing said transfected or transduced cells, and
c) administering a substrate of the herpes simplex virus thymidine kinase protein and an icaspase9 protein activator simultaneously to the culture when the cells cultured in step (b) are to be eliminated.

Another aspect of the invention relates to a method for inducing cell death in a specific cell population within an individual; or method for treating pathologies, diseases, syndromes, disorders, pathological or physiological conditions with the unwanted presence and/or proliferation of certain cells; or method for treating graft-versus-host disease, transplant rejection or a tumor, preferably solid, more preferably cancer, in an individual, wherein said individual is preferably human, comprising:
a) introducing the gene construct or the expression vector of the invention in the target cells targeted for elimination, preferably by means of one or more specifically directed vectors, wherein more preferably said target cells are cells previously administered during a transplant or tumor cells, and
b) administer a substrate of the herpes simplex virus thymidine kinase protein and an icaspase9 protein activator simultaneously.

Alternatively, to step (a) of the method described in the preceding paragraph, cells previously transfected or transduced with the gene construct or expression vector of the invention can be introduced, i.e. the cell or cell population of the invention, in the vicinity of the tumor or transplant.

Throughout the description and the claims, the word "comprises" and its variants are not intended to exclude other technical characteristics, additives, components or steps. For persons skilled in the art, other objects, advantages and characteristics of the invention may be deduced from both the description and the embodiment of the invention. The following examples and figures are provided by way of example and are not intended to limit the present invention.

### DESCRIPTION OF THE FIGURES

**Fig. 1****. Shows the structure of two preferred polycistronic suicide cassettes of the invention.** Two possible versions of the suicide construct, the first with the mCherry (pLF1a-Suicide(Hygro)2.0) protein and the second with the ΔNGFR (pSuicide-OL-ΔNGFR) receptor as marker genes, are shown.

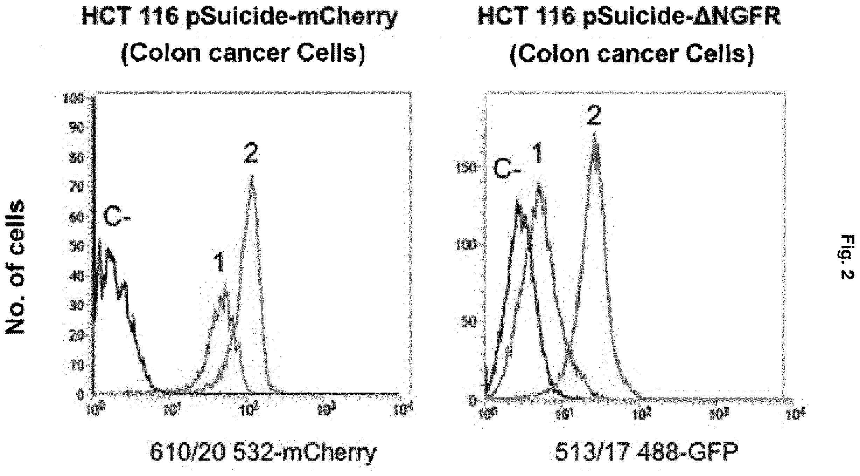

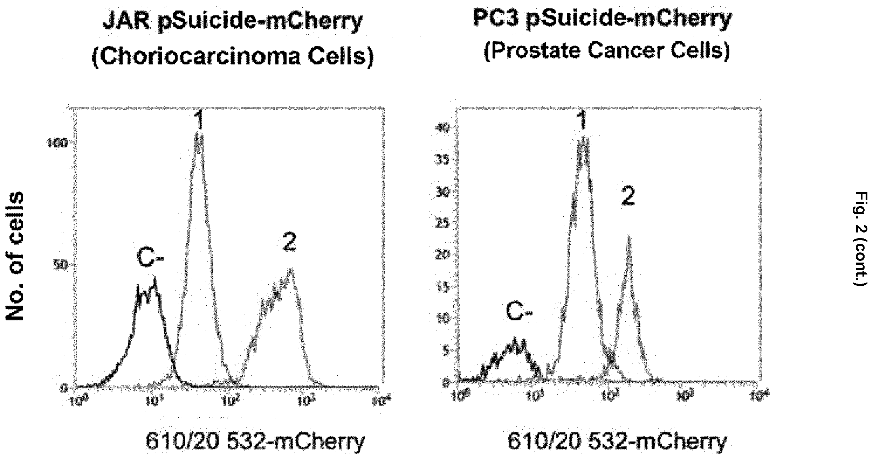

**Fig. 2****. Shows the characterization by FACS of the suicide construct-bearing cell lines in the three types of assayed tumor cells.** The number of cells is represented on the y-axis, the intensity of the *m-Cherry* fluorescent signal or FITC in the case of ΔNGFR is represented on the x-axis. The corresponding untransfected cell types were used as a control. The monoclonal lines are distributed in accordance with their red or green fluorescence intensity. The negative control (C-), a clone with a low expression of the construct (1) and a clone with a high expression of the construct (2) are represented for each cell line.

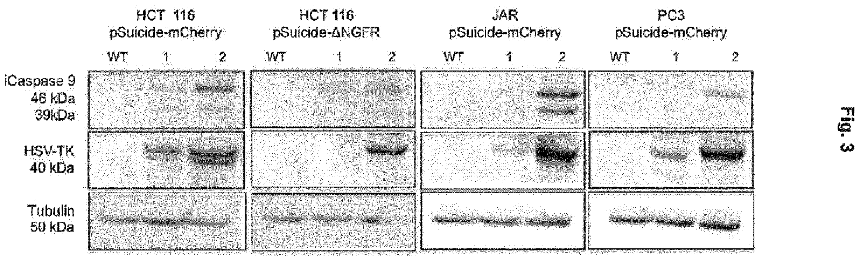

**Fig. 3****. Shows the characterization of the transgenic suicide protein expression in the monoclonal populations of each cell type.** The expression of the two suicide proteins was analyzed: iCaspase 9 (46kDa the inactive form and 39kDa the active form) and 40kDa HSV-TK in the absence of dimerizer and Ganciclovir. Tubulin (50kDa) was used as a load control. A negative control was included in each untransfected cell line (WT), a clone with a low expression of the suicide construct (1) and a clone with a high expression of the suicide construct (2).

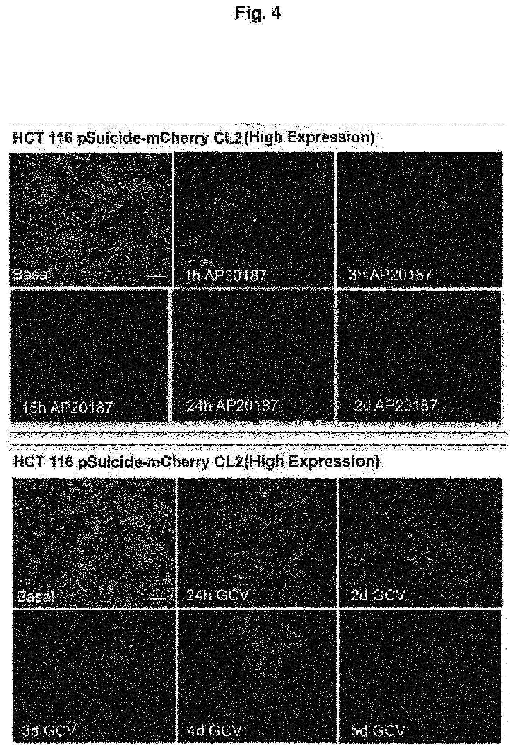

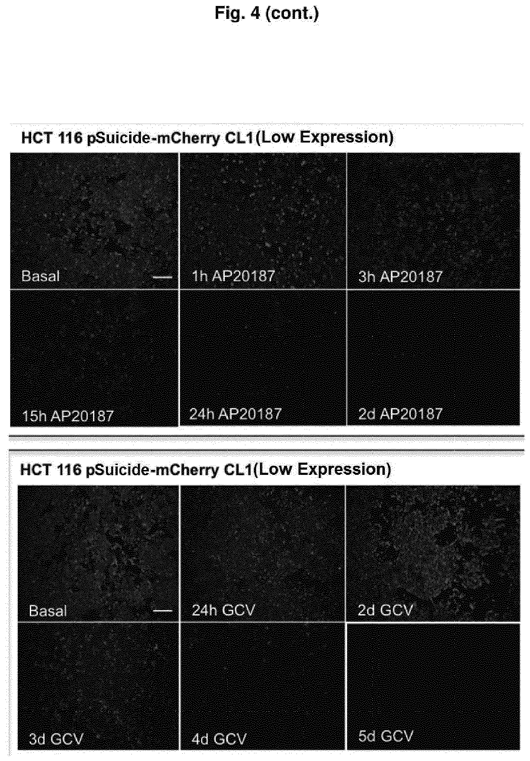

**Fig. 4****. Shows the cytotoxicity mediated by AP20187 (100 nM) and GCV (10 µM) in HCT116 cells transfected with the mCherry suicide cassette.** Fluorescence microscopy in GFP, with a 5x lens, in HCT 116 cells marked with *Calcein Green* and cultured with the corresponding treatment for the indicated time periods. The scale bar corresponds to 400 µm.

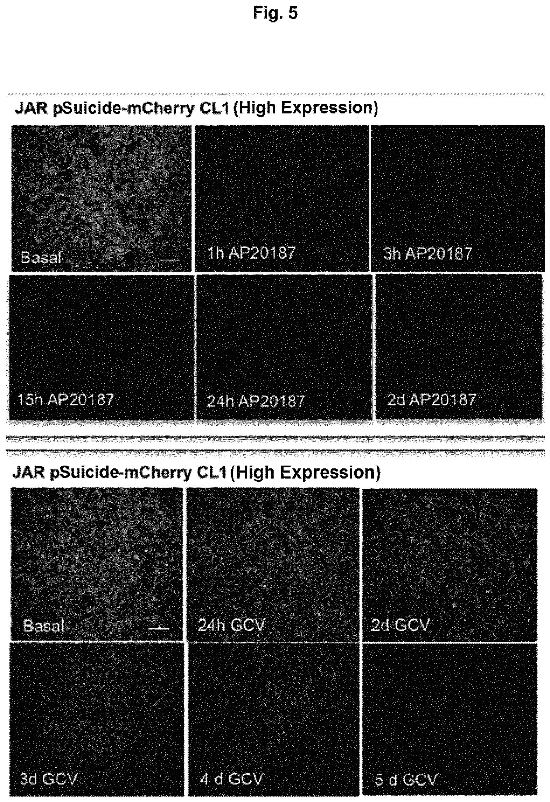

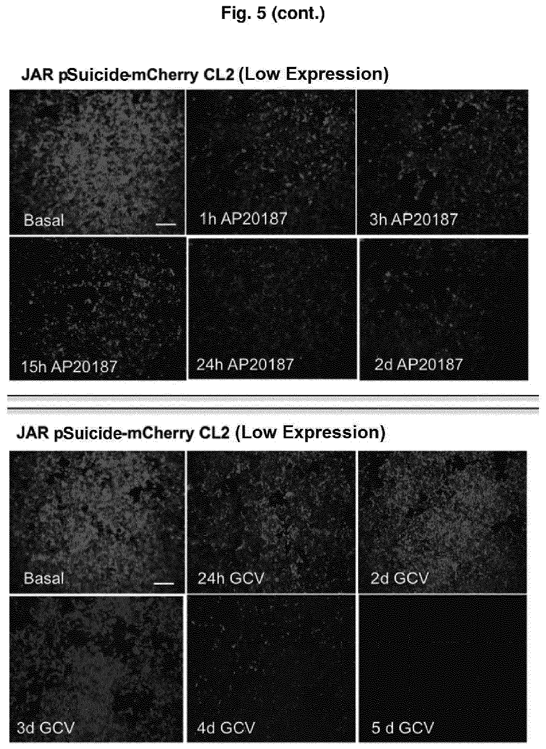

**Fig. 5****. Shows the cytotoxicity mediated by AP20187 (100 nM) and GCV (10 µM) in JAR cells transfected with the mCherry suicide cassette.** Fluorescence microscopy in GFP, with a 5x lens, in JAR cells marked with *Calcein Green* and cultured with the corresponding treatment for the indicated time periods. The scale bar corresponds to 400 µm.

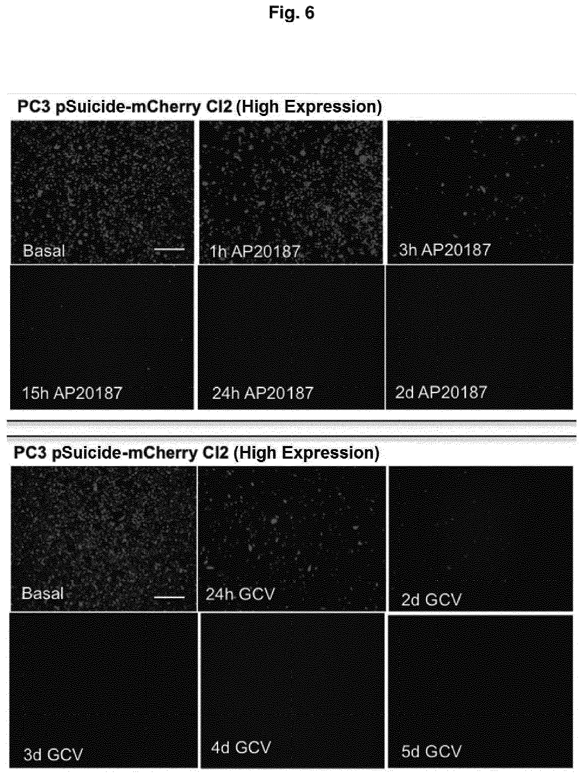

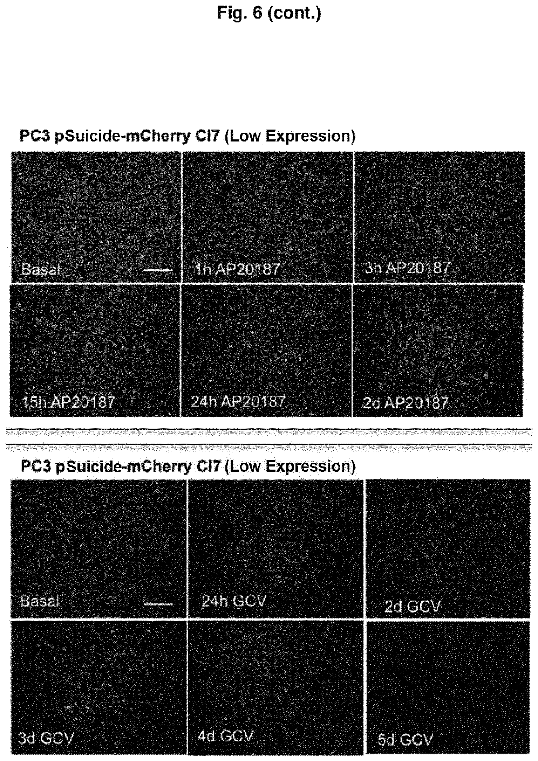

**Fig. 6****. Shows the cytotoxicity mediated by AP20187 (100 nM) and GCV (10 µM) in PC3 cells transfected with the mCherry suicide cassette.** Fluorescence microscopy in GFP, with a 5x lens, in PC3 cells marked with *Calcein Green* and cultured with the corresponding treatment for the indicated time periods. The scale bar corresponds to 400 µm.

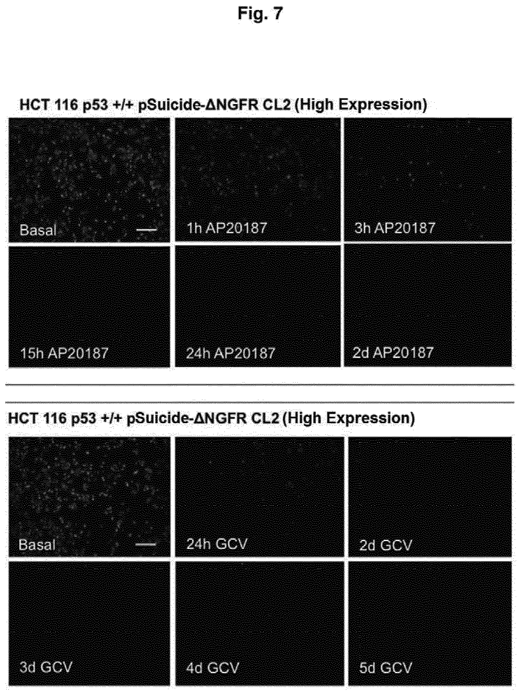

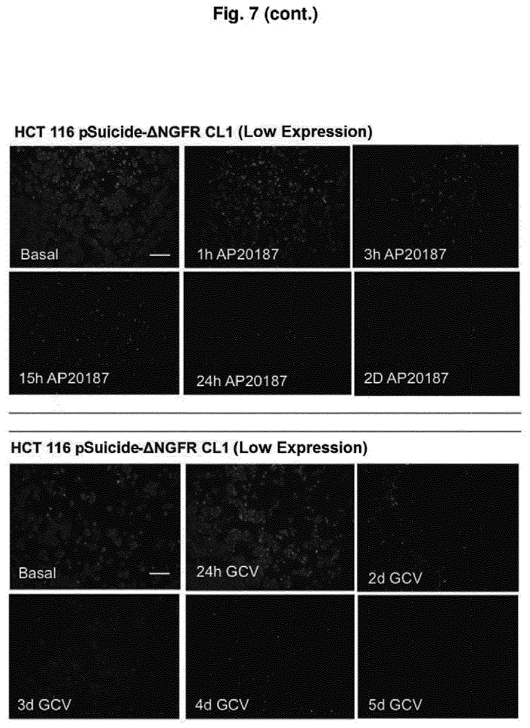

**Fig. 7****. Shows the cytotoxicity mediated by AP20187 (100 nM) and GCV (10 µM) in HCT cells transfected with the ΔNGFR suicide cassette.** Fluorescence microscopy in GFP, with a 5x lens, in HCT 116 cells marked with *Calcein Green* and cultured with the corresponding treatment for the indicated time periods. The scale bar corresponds to 400 µm.

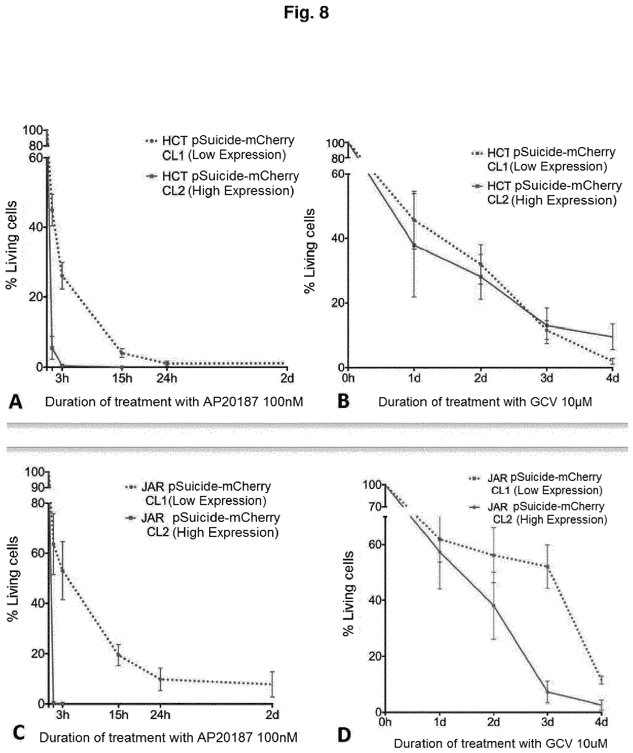

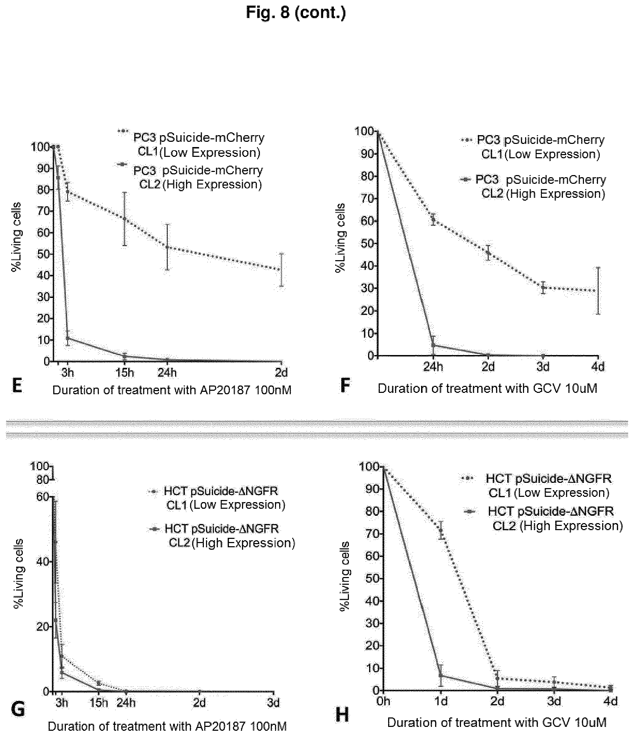

**Fig. 8****. Shows the quantification of the cytotoxicity mediated by AP20187 and GCV in the different clones of the three cell lines: HCT, JAR and PC3.** The data obtained after the quantification of the microscopy images are represented using the QWin program. The percentage of living cells is represented on the y-axis. The duration of the treatment is represented on the x-axis. A-B) HCT 116 pSuicide-mCherry; C-D) JAR pSuicide-mCherry; E-F) PC3 pSuicide-mCherry and G-H) HCT 116 pSuicide-ΔNGFR. In all cases, the clone with high expression of the construct is represented by a solid line and the clone with low expression of the construct is represented with a dashed line. Each point represents the average of 4 repetitions.
**Fig. 9****. Shows the number of colonies resistant to the different treatments.** Clones of the HCT 116, JAR and PC3 lines with high or low expression of the pSuicide-mCherry vector were treated for 10 days with AP20187 100 nM, GCV 10µM or both simultaneously.

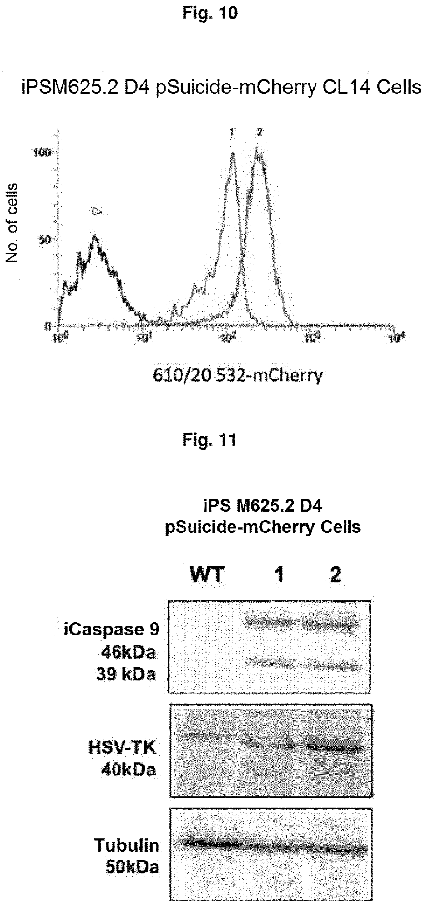

**Fig. 10****. Shows the characterization by FACS of clones of suicide construct-bearing mouse iPS cells.** The number of cells is represented on the y-axis, the intensity of the mCherry fluorescent signal is represented on the x-axis. The same untransfected cell line was used as a control. The *Wild Type* negative control (C-), a low expression clone (1) and a high expression clone (2) are shown.
**Fig. 11****. Shows the characterization of the constitutive expression of the transgenic suicide proteins in the monoclonal populations of iPS cells using Western Blot.** The expression of the two suicide proteins was analyzed: iCaspase 9 (46kDa the inactive form and 39kDa the active form) and 40kDa HSV-TK. Tubulin (50kDa) was used as a load control. A WT negative control, a clone with a low expression of the suicide construct (1) and a clone with a high expression (2) were included.

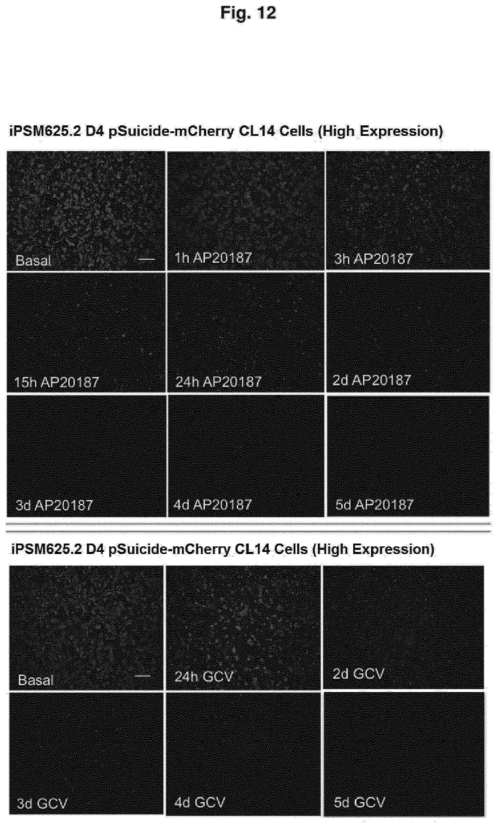

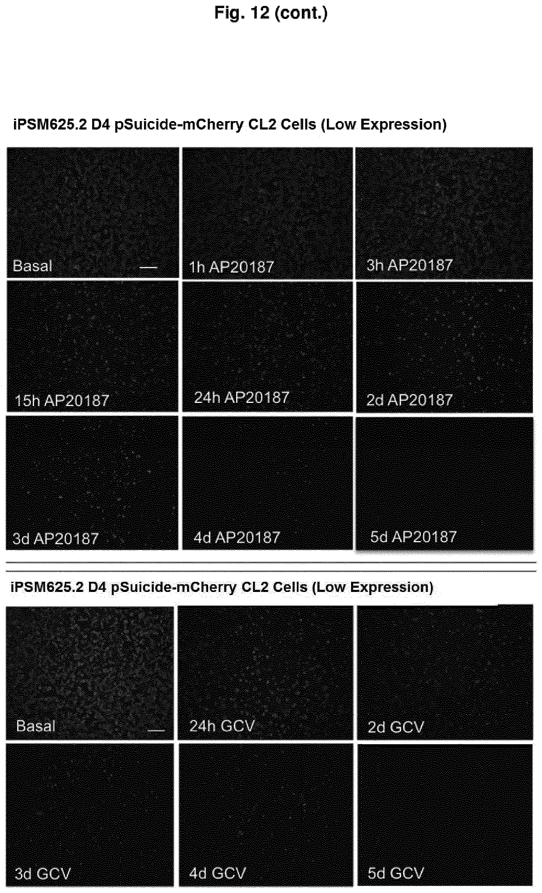

**Fig. 12****. Shows the cytotoxicity mediated by AP20187 (100 nM) and GCV (10 µM) in mouse iPS cells transfected with the mCherry suicide cassette.** Fluorescence microscopy in GFP, with a 5x lens, of M625.2 D4 cells marked with *Calcein Green* and cultured with the corresponding treatment. The scale bar corresponds to 400 µm.

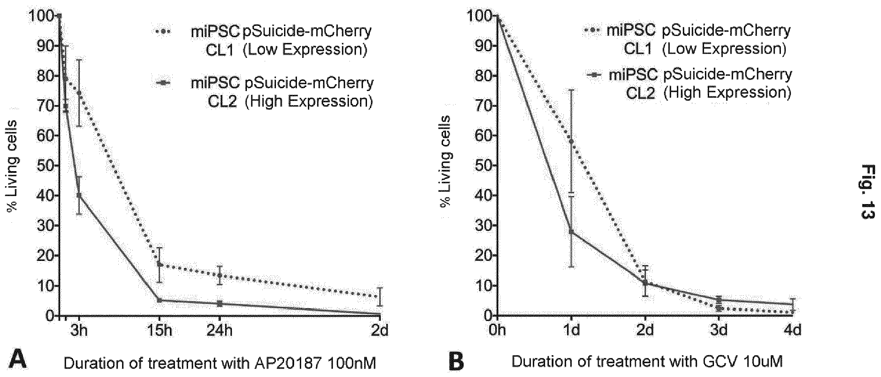

**Fig. 13****. Shows the quantification of the cytotoxicity mediated by AP20187 and GCV in the two clones of the mouse iPS cells.** The data obtained after the quantification of the microscopy images are represented using QWin. Cell viability (y-axis) is represented in accordance with the duration of the corresponding treatment (x-axis). In A) Cells treated with AP20187 and in B) Cells treated with GCV. In all cases, the clone with high expression of the construct is represented by a solid line and the clone with low expression of the construct is represented with a dashed line. Each point represents the average of 4 samples.

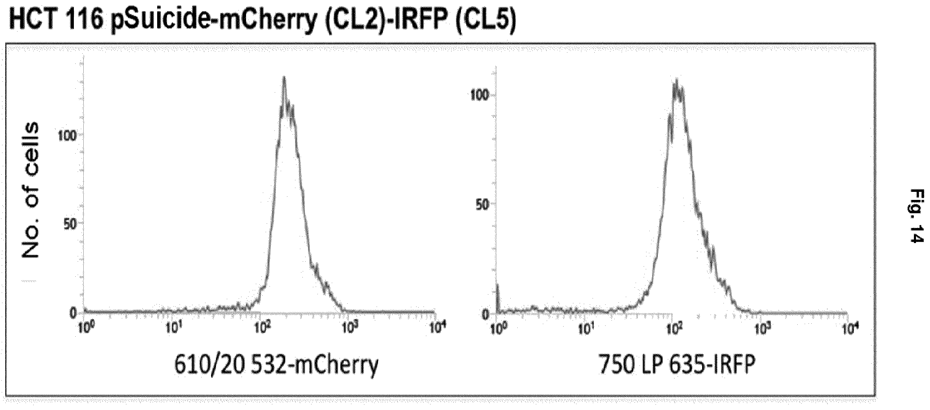

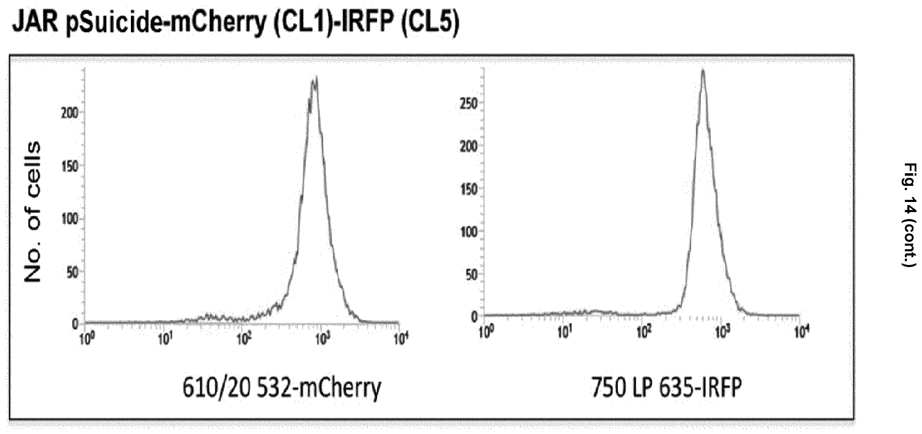

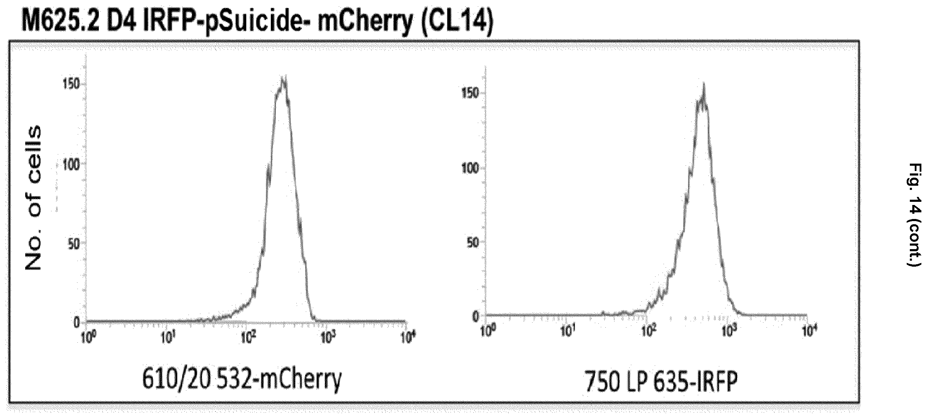

**Fig. 14****. Shows the expression levels of the mCherry protein and of the IRFP vector in the three clones selected for the *in vivo* assays.** The expression levels of the two markers were analyzed by FACS. Double positives were selected to perform *in vivo* transplants.
**Fig. 15****. Shows the variation in size of teratomas and tumors during treatment with AP20187 and GCV.** The days elapsed since the injection of the cells in mice are represented on the x-axis. The size of the tumors is represented on the y-axis in mm³. The short arrows on a solid line indicate the administration of GCV at a rate of 1.2 mg by injection (50 mg/kg). The long arrows on a dashed line indicate the administration of AP20187 at a rate of 50 µg per injection (2 mg/kg).

### EXAMPLES

The invention is illustrated below by means of assays conducted by the inventors, which demonstrate the effectiveness of the gene construct of the invention or of the composition of the invention in the selective and directed elimination of target cell populations.

### EXAMPLE 1. EFFECT OF THE SUICIDE CASSETTE IN TUMOR CELL LINES.

In order to verify the proper functioning of the F1a-Suicide (Hygro)2.0 suicide cassette, it was transfected in three tumor cell lines: PC3 (human prostate carcinoma), JAR (Human choriocarcinoma) and HCT 116 (human colon carcinoma).

Once mCherry constitutive expression lines were obtained from each of the original tumor lines, the fluorescent protein expression was analyzed by *"Fluorescence-Activated Cell Sorting"* (FACS). The expression levels of the construct, estimated based on mCherry fluorescence, were found to vary from one line to another, as in the case of the treatments with Ganciclovir (GVC) and with the dimerizer AP20187 (Table 1).

**Table 1. Cell death observed in cell populations with treatments of varying duration with 100 nM of dimerizer (AP20187) or 10µM Ganciclovir (GCV). Staurosporin (STS) was used as a positive cell death induction control.**

| **CELL LINE** | **3 HOURS** | | | **24 HOURS** | | | **54 HOURS** | | |
|---|---|---|---|---|---|---|---|---|---|
| | **STS** | **AP2018 7** | **GC V** | **STS** | **AP2018 7** | **GC V** | **STS** | **AP2018 7** | **GC V** |
| **HCT 116 pSuicide** - | 90 % | 70 % | 0 % | 100 % | 50 % | 0 % | 100 % | 20 % | 15 % |
| **mCherry 1** | | | | | | | | | |
| **HCT 116 pSuicide - mCherry 2** | 100 % | 80 % | 0 % | 100 % | 70 % | 0 % | 100 % | 20 % | 10 % |
| **JAR pSuicide - mCherry 1** | 70 % | 30 % | 0 % | 100 % | 30 % | 0 % | 100 % | 10 % | 0 % |
| **JAR pSuicide - mCherry 2** | 70 % | 100 % | 0 % | 100 % | 90 % | 0 % | 100 % | 90 % | 0 % |

The most plausible explanation for these results is that, due to the polyclonal nature of the lines obtained from the transfection, each was composed by different populations that expressed the suicide proteins at different levels. Consequently, their mortality was very heterogeneous, varying both between the lines and between the different clones constituting each of them.

Based on these first results, it was decided to isolate, in each line, cell clones with high and low expression of the construct in order to obtain a cleaner system in which to accurately study the effectiveness of the suicide cassettes (Fig. 2). The clones were isolated by FACS in accordance with the levels of fluorescence emitted by the last element of the tricistronic messenger: the m-Cherry protein in the case of pLF1a-Suicide (Hygro)2.0, or by staining with an anti- ΔNGFR antibody conjugated with FITC in the case of pSuicide-OL-ΔNGFR.

Once the clones have been isolated and their monoclonality demonstrated, the basal expression of the iCaspase 9 and TK proteins was verified by Western Blot (Fig. 3).

The results revealed a clear correlation between the relative levels of the suicide proteins and fluorescence readings obtained by FACS, and demonstrated the efficiency of the 2A elements in the production of more than one protein from a single promoter. After establishing the proper functioning of the designed constructs, their effectiveness as cell death-inducing tools was evaluated. To this end, the different clones of each cell line were treated with 100 nM of AP20187 and 10µM of GCV.

In this assay, the cells of the selected clones were sown on 96-well plates. The study of cell death due to the different treatments was based on staining with *Calcein Green,* a cell staining agent that fluoresces green only in living cells. The images obtained using a Leica DMI6000B microscope (Figs. 4-7), were analyzed and quantified using the Leica QWin program (Fig. 8). The program measured the surface of each well that remained covered with cells at different treatment times with each of the two cell death inducers.

Three main conclusions can be drawn from the analysis of the results. The first, observing the activity of the iCaspase9 activity (treatment with AP20187), is that a lower level of expression of the suicide cassette is correlated with a reduced response to induced death. This indicates that the amount of iCasp9 protein present in the cell constitutively is essential to the functioning of the suicide system, and that this data must be seriously taken into account in clinical application. In the three cell lines, treatments of between 1 and 3 hours with the dimerizer suffice to eliminate the cells of clones with high expression of the suicide cassette, but only 50% in the case of cells of clones of the same cell type with a low expression level.

The second conclusion is that the effect of administering GCV is felt much later than that of administering AP20187. For example, GCV must be administered for at least 24 hours so that approximately 50% of the cells of clones with a high expression level die. The differences observed between the two treatments are due mainly to the fact that the corresponding suicide proteins act in two different systems: induction of cell death by apoptosis in the case of AP20187 and induction of death by DNA damage-induced senescence in the case of GCV. Additionally, in the case of the TK, such a clear difference between clones with high and low expression such as that observed with iCaspase is not detected. This can be clearly observed in HCT pSuicide-mCherry, wherein the difference in expression between the high and low expression clones is not very large.

The third is that the cells are not eliminated in their entirety by applying separate *in vitro* treatments with AP20187 or GCV. There is a percentage of cells that for some reason develop resistance to the treatment.

It is precisely these resistant cells which are the main target of the suicide cassette of the invention. In order to explore their behavior, a study was carried out with the three cell lines, wherein the number of resistant colonies obtained per each million cells sown after 10 days of treatment with the dimerizer AP20187, GCV or both simultaneously were counted manually. The experiment was extended for the purpose of observing the evolution of the resistant cells in the long term.

Once again, the difference already detected between high and low expression clones was observed. Resistant cell colonies did not appear in the first, whereas in the second the results indicated that the number of resistant colonies obtained after the double treatment was lower than with two treatments separately (Fig. 9). In this experiment a single administration was made at the start of the treatment. In the case of the double treatment, both compounds were added simultaneously. Taking into account that in clinical practice it is not always possible to select high expression clones, the existence of a mechanism that reduces the risk of appearance of cell colonies resistant to the treatment represents a significant increase in system safety.

### EXAMPLE 2. TRANSFECTION IN iPS CELLS.

After verifying *in vitro* the proper functioning of the new suicide cassette in tumor cell lines, the next step was its application to induced pluripotent stem cells (iPS). A mouse iPSC line was used (M625.2 D4). The iPSC cells were transfected with the pSuicide-mCherry cassette, whose expression levels were analyzed by FACS (Fig. 10). This time the high and low expression clones were directly isolated in accordance with the fluorescence levels of the mCherry protein. In this case, anticipating the *in vivo* studies, the iPS cells were also transfected with an infrared fluorescent protein expression vector (pIRV3), such that the positive double clones, IRFP and mCherry, were detectable once transplanted to an animal.

The characterization of the two clones of mouse iPS cells was completed with a study of the constitutive expression levels of the transgenic suicide proteins using Western Blot (Fig. 11).

After establishing the two cell lines as stable lines that express the suicide construct, an *in vitro* assay on 96-well plate was conducted to verify the effectiveness of the system following the previously detailed diagram for tumor cells. Figure 12 shows the images taken by *in vivo* fluorescence microscopy. Figure 13 shows the quantification of the same images using the Leica QWin program.

In this manner, it was verified that the suicide system functions both in tumor cells and in induced pluripotent stem cells. Once again, it was observed that the treatment with the dimerizer has a faster effect than the GCV, although the iPS cells seem to have greater sensitivity to the GCV.

### EXAMPLE 3. IN VIVO ASSAY: TRANSFECTED CELL TRANSPLANT.

As a whole, the results obtained *in vivo* demonstrated the functionality of the construct, both in the different human tumor cell lines and in the iPS murine cells. Also, they demonstrated the correlation between the basal expression level of the suicide construct and its effectiveness in the elimination of the carrier cells. Lastly, it was observed that the double treatment was more effective than the other two treatments separately in preventing the appearance of resistant colonies.

In order to verify the validity of these observations and the possible application of the new construct in clinical practice, their effectiveness in *in vivo* experiments was subsequently determined.

The main objective of this assay was to verify the possibility of selectively eliminating previously transplanted cells. Three of the four stable cell lines generated were selected and characterized in the previous examples: HCT 116, JAR and M625.2 D4.

In an *in vivo* experiment, the exposure of the cells to the treatment is much lower than that achieved *in vitro.* Often, the blood supply conditions and the complexity of the three-dimensional structure in which they are located hamper access of the assayed compounds to the target tissue or cell. In order to establish the most restrictive possible conditions when establishing comparisons between the effectiveness of the individual treatments with GCV or AP20187 and the combined treatment, GCV+AP20187, it was decided to use the high expression clones, which in the *in vitro* experiments practically did not exhibit any differences therebetween. The experiments were conducted using the human HCT 116 and JAR cell lines and the mouse iPS M625.2 D4 cell line. An infrared vector (pIRV3) was introduced in the human lines in order to monitor the transplanted cells. Fig. 14 shows the infrared expression levels in each of the selected clones.

*Nude* Foxn1^{nu/nu} mice irradiated with 4G were used as receptors to cause immunosuppression symptoms. The cells were injected subcutaneously (100µl) in the flanks of the receptors, at a rate of 1×10⁶ cells per injection. A total of 24 mice were used per line, divided into 4 experimental groups: controls (injected with cell-free medium, (PBS)), animals injected with cells and treated only with AP20187 (1 mg/kg, dissolved in 4% ethanol, 10% PEG-400 and 86% PBS-2% Tween), animals injected with cells and treated only with GCV (50 mg/kg in doses of 12.5 mg/ml in PBS every 2 days), and animals injected with cells and treated simultaneously with AP20187 and GCV. The treatments were administrated by intraperitoneal injection after verifying the appearance of tumors. The tumors were measured every two days. Figure 15 shows the graphs that include the variation in the size of the tumors (teratoma in the case of the iPS cells) during the treatments.

Both treatment with AP20187 and with GCV delayed the growth of the tumors, but what is really important is that the combined treatment with the two reagents improved each separately, completely stopping tumor growth, and even reducing it in some cases.

It is important to take into account that the effectiveness of the *in vivo* treatment depends on many factors such as access of the tumor to the blood stream, the dose, route of administration, etc. Modifying these variables will make it possible to significantly improve the effectiveness of the treatment.

## Claims

1. A polycistronic gene construct comprising:
a) a gene promoter, preferably of eukaryotic origin,
b) an intron, preferably that of beta-globin,
c) an encoding nucleotide sequence for the icaspase9 protein,
d) a first translation initiation or continuation element, preferably of the 2A type,
e) an encoding nucleotide sequence for the herpes simplex virus thymidine kinase protein,
f) a second translation initiation or continuation element, preferably of the 2A type, and
g) preferably at least one marker gene.

2. The gene construct according to claim 1, wherein elements (a) to (g) are disposed in the 5' to 3' sense in said gene construct.

3. An expression vector comprising the gene construct according to any of claims 1 or 2.

4. An isolated cell or cell population comprising the gene construct according to any of claims 1 or 2, or the expression vector according to claim 3.

5. The cell or cell population according to claim 4, wherein said cell or cell population is eukaryotic, preferably human.

6. The cell or cell population according to any of claims 4 or 5, wherein said cell or cell population is a cell or cell population of the immune system, preferably a T-lymphocyte or population of T-lymphocytes.

7. The cell or cell population according to any of claims 4 or 5, wherein said cell or cell population is a tumoral or a stem cell or stem cell population.

8. A composition comprising:
i. the gene construct according to any of claims 1 or 2, the expression vector according to claim 3, or the cell or cell population according to any of claims 4 to 7,
ii. a substrate of the herpes simplex virus thymidine kinase protein, and
iii. an icaspase9 protein activator.

9. The composition according to claim 8, wherein the substrate of the herpes simplex virus thymidine kinase protein is Ganciclovir, Aciclovir, AZT, Stavudine, Idoxuridine or FIAU (1-(2-deoxy-2-fluoro-1-D-arabinofuranoside)-5-iodouracil), preferably Ganciclovir.

10. The composition according to any of claims 8 or 9, wherein the icaspase9 protein activator is AP20187 or AP1903, preferably AP20187.

11. The composition according to any of claims 8 to 10, which is a pharmaceutical composition, wherein said pharmaceutical composition preferably further comprises a pharmaceutically acceptable carrier.

12. The gene construct according to any of claims 1 or 2, the expression vector according to claim 3, the cell or cell population according to any of claims 4 to 7, or the composition according to any of claims 8 to 11, for use as a medicament.

13. The gene construct according to any of claims 1 or 2, the expression vector according to claim 3, the cell or cell population according to any of claims 4 to 7, or the composition according to any of claims 8 to 11, for use in the treatment and/or prevention of tumors, graft-versus-host disease or transplant rejection.

14. The composition according to any of claims 8 to 11, for use according to any of claims 12 or 13, wherein the substrate of the herpes simplex virus thymidine kinase protein and the icaspase9 protein activator are administered simultaneously.

15. An *in vitro* method for obtaining cells useful for a transplant comprising:
a) transfecting the cells to be transplanted with the gene construct according to any of claims 1 or 2, or with the expression vector according to claim 3, and
b) culturing said transfected cells to obtain a sufficient number for a transplant.

## Patentansprüche

1. Polycistronisches Genkonstrukt, umfassend:
a) einen Genpromotor, vorzugsweise eukaryontischen Ursprungs,
b) ein Intron, vorzugsweise das von Beta-Globin,
c) eine kodierende Nukleotidsequenz für das Icaspase9-Protein,
d) ein erstes Translationsinitiierungs- oder -fortsetzungselement, vorzugsweise vom Typ 2A,
e) eine kodierende Nukleotidsequenz für das Thymidinkinase-Protein des Herpes-Simplex-Virus,
f) ein zweites Translationsinitiierungs- oder -fortsetzungselement, vorzugsweise vom Typ 2A, und
g) vorzugsweise mindestens ein Markergen.

2. Genkonstrukt gemäß Anspruch 1, wobei die Elemente (a) bis (g) in dem Genkonstrukt in der 5'- bis 3'-Richtung angeordnet sind.

3. Expressionsvektor, umfassend das Genkonstrukt gemäß einem der Ansprüche 1 oder 2.

4. Isolierte Zelle oder Zellpopulation, umfassend das Genkonstrukt gemäß einem der Ansprüche 1 oder 2 oder den Expressionsvektor gemäß Anspruch 3.

5. Zelle oder Zellpopulation gemäß Anspruch 4, wobei die Zelle oder Zellpopulation eukaryontisch, vorzugsweise menschlich, ist.

6. Zelle oder Zellpopulation gemäß einem der Ansprüche 4 oder 5, wobei die Zelle oder Zellpopulation eine Zelle oder Zellpopulation des Immunsystems, vorzugsweise ein T-Lymphozyt oder eine Population von T-Lymphozyten, ist.

7. Zelle oder Zellpopulation gemäß einem der Ansprüche 4 oder 5, wobei die Zelle oder Zellpopulation eine Tumorzelle oder eine Stammzelle oder Stammzellpopulation ist.

8. Zusammensetzung, umfassend:
i. das Genkonstrukt gemäß einem der Ansprüche 1 oder 2, den Expressionsvektor gemäß Anspruch 3 oder die Zelle oder Zellpopulation gemäß einem der Ansprüche 4 bis 7,
ii. ein Substrat des Thymidinkinase-Proteins des Herpes-simplex-Virus und
iii. einen Icaspase9-Proteinaktivator.

9. Zusammensetzung gemäß Anspruch 8, wobei das Substrat des Thymidinkinase-Proteins des Herpes-simplex-Virus Ganciclovir, Aciclovir, AZT, Stavudin, Idoxuridin oder FIAU (1-(2-Desoxy-2-fluor-1-D-arabinofuranosid)-5)-ioduracil), vorzugsweise Ganciclovir, ist.

10. Zusammensetzung gemäß einem der Ansprüche 8 oder 9, wobei der Icaspase9-Proteinaktivator AP20187 oder AP1903, vorzugsweise AP20187, ist.

11. Zusammensetzung gemäß einem der Ansprüche 8 bis 10, bei der es sich um eine pharmazeutische Zusammensetzung handelt, wobei die pharmazeutische Zusammensetzung vorzugsweise außerdem einen pharmazeutisch verträglichen Träger umfasst.

12. Genkonstrukt gemäß einem der Ansprüche 1 oder 2, Expressionsvektor gemäß Anspruch 3, Zelle oder Zellpopulation gemäß einem der Ansprüche 4 bis 7 oder Zusammensetzung gemäß einem der Ansprüche 8 bis 11 zur Verwendung als Medikament.

13. Genkonstrukt gemäß einem der Ansprüche 1 oder 2, Expressionsvektor gemäß Anspruch 3, Zelle oder Zellpopulation gemäß einem der Ansprüche 4 bis 7 oder Zusammensetzung gemäß einem der Ansprüche 8 bis 11 zur Verwendung bei der Behandlung und/oder Vorbeugung von Tumoren, Graft-versus-Host-Krankheit oder Transplantatabstoßung.

14. Zusammensetzung gemäß einem der Ansprüche 8 bis 11 zur Verwendung gemäß einem der Ansprüche 12 oder 13, wobei das Substrat des Thymidinkinase-Proteins des Herpes-simplex-Virus und der Icaspase9-Proteinaktivator gleichzeitig verabreicht werden.

15. In-vitro-Verfahren zur Gewinnung von Zellen, die für eine Transplantation nützlich sind, umfassend:
a) Transfektion der zu transplantierenden Zellen mit dem Genkonstrukt gemäß einem der Ansprüche 1 oder 2 oder mit dem Expressionsvektor gemäß Anspruch 3 und
b) Kultivieren der transfizierten Zellen, um eine ausreichende Anzahl für eine Transplantation zu gewinnen.

## Revendications

1. Construction génique polycistronique comprenant :
a) un promoteur de gène, de préférence d'origine eucaryote,
b) un intron, de préférence celui de la bêta-globine,
c) une séquence nucléotidique codante pour la protéine icaspase9,
d) un premier élément d'initiation ou de continuation de traduction, de préférence du type 2A,
e) une séquence nucléotidique codante pour la protéine thymidine kinase du virus Herpes simplex,
f) un second élément d'initiation ou de continuation de traduction, de préférence du type 2A, et
g) de préférence au moins un gène marqueur.

2. Construction génique selon la revendication 1, les éléments (a) à (g) étant disposés dans le sens de 5' vers 3' dans ladite construction génique.

3. Vecteur d'expression comprenant la construction génique selon l'une quelconque des revendications 1 ou 2.

4. Cellule isolée ou population de cellules comprenant la construction génique selon l'une quelconque des revendications 1 ou 2 ou le vecteur d'expression selon la revendication 3.

5. Cellule ou population de cellules selon la revendication 4, ladite cellule ou population de cellules étant eucaryote, de préférence humaine.

6. Cellule ou population de cellules selon l'une quelconque des revendications 4 ou 5, ladite cellule ou population de cellules étant une cellule ou population de cellules du système immunitaire, de préférence un lymphocyte T ou une population de lymphocytes T.

7. Cellule ou population de cellules selon l'une quelconque des revendications 4 ou 5, ladite cellule ou population de cellules étant une cellule tumorale ou souche ou une population de cellules souches.

8. Composition comprenant :
i. la construction génique selon l'une quelconque des revendications 1 ou 2, le vecteur d'expression selon la revendication 3 ou la cellule ou population de cellules selon l'une quelconque des revendications 4 à 7,
ii. un substrat de la protéine thymidine kinase du virus Herpes simplex et
iii. un activateur de la protéine icaspase9.

9. Composition selon la revendication 8, dans laquelle le substrat de la protéine thymidine kinase du virus Herpes simplex est le ganciclovir, l'aciclovir, l'AZT, la stavudine, l'idoxuridine ou le FIAU (1-(2-désoxy-2-fluoro-1-D-arabinofuranoside)-5-iodouracile), de préférence le ganciclovir.

10. Composition selon l'une quelconque des revendications 8 ou 9, dans laquelle l'activateur de la protéine icaspase9 est AP20187 ou AP1903, de préférence AP20187.

11. Composition selon l'une quelconque des revendications 8 à 10, qui est une composition pharmaceutique, ladite composition pharmaceutique comprenant en outre de préférence un véhicule pharmaceutiquement acceptable.

12. Construction génique selon l'une quelconque des revendications 1 ou 2, vecteur d'expression selon la revendication 3, cellule ou population de cellules selon l'une quelconque des revendications 4 à 7 ou composition selon l'une quelconque des revendications 8 à 11, pour une utilisation en tant que médicament.

13. Construction génique selon l'une quelconque des revendications 1 ou 2, vecteur d'expression selon la revendication 3, cellule ou population de cellules selon l'une quelconque des revendications 4 à 7 ou composition selon l'une quelconque des revendications 8 à 11, pour une utilisation dans le traitement et/ou la prévention de tumeurs, de la maladie du greffon contre l'hôte ou du rejet de greffe.

14. Composition selon l'une quelconque des revendications 8 à 11, pour une utilisation selon l'une quelconque des revendications 12 ou 13, le substrat de la protéine thymidine kinase du virus Herpes simplex et l'activateur de la protéine icaspase9 étant administrés simultanément.

15. Procédé *in vitro* d'obtention de cellules utiles pour une greffe comprenant :
a) la transfection des cellules à greffer avec la construction génique selon l'une quelconque des revendications 1 ou 2 ou avec le vecteur d'expression selon la revendication 3 et
b) la culture desdites cellules transfectées pour obtenir un nombre suffisant pour une greffe.
